(19) Europäisches Patentamt
European Patent Office
Office européen des brevets

(11) Veröffentlichungsnummer: **0 002 450**
**B1**

(12) # EUROPÄISCHE PATENTSCHRIFT

(45) Veröffentlichungstag der Patentschrift: **20.05.81**

(21) Anmeldenummer: **78101397.4**

(22) Anmeldetag: **18.11.78**

(51) Int. Cl.³: **C 07 H 15/22, A 61 K 31/70 //C07D333/48, C07D207/48, C07H13/12, C07D211/46, C07D409/12, C07C145/02, C07C79/26, C07C93/16**

(54) **1-N-4,6-Di-O-(aminoglykosyl)-1,3-diamino-cyclitol-Derivate, Verfahren zu ihrer Herstellung sowie sie enthaltende Arzneimittel.**

(30) Priorität: **02.12.77 DE 2753769**

(43) Veröffentlichungstag der Anmeldung: **27.06.79 Patentblatt 79/13**

(45) Bekanntmachung des Hinweises auf die Patenterteilung: **20.05.81 Patentblatt 81/20**

(84) Benannte Vertragsstaaten: **BE CH DE FR GB NL SE**

(56) Entgegenhaltungen: **FR - A - 2 264 553**

(73) Patentinhaber: **BAYER AG**
**Zentralbereich Patente, Marken und Lizenzen**
**D-5090 Leverkusen 1, Bayerwerk (DE)**

(72) Erfinder: **Petersen, Uwe, Dr.**
**Auf dem Forst 4**
**D-5090 Leverkusen 1 (DE)**
Erfinder: **Voss, Eckart, Dr.**
**Morgengraben 10**
**D-5000 Köln 80 (DE)**
Erfinder: **Metzger, Karl Georg, Dr.**
**Pahlkestrasse 15**
**D-5600 Wuppertal 1 (DE)**
Erfinder: **Stadler, Peter, Dr.**
**Ohligserstrasse 85**
**D-5657 Haan (DE)**

EP 0 002 450 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# 1-N-4,6-Di-O-(aminoglykosyl)-1,3-diamino-cyclitol-Derivate, Verfahren zu ihrer Herstellung sowie sie enthaltende Arzneimittel

Die Erfindung betrifft 1-N-4,6-Di-O-(aminoglykosyl)-1,3-diamino-cyclitol-Derivate, Verfahren zu ihrer Herstellung und ihre Verwendung als Arzneimittel, insbesondere als antimikrobielle Mittel.

Aminoglykosid-Antibiotika sind wichtige Substanzen zur wirkungsvollen Bekämpfung bakterieller Infektionen. Das Auftreten resistenter Keime mindert jedoch in vielen Fällen ihre breite Anwendbarkeit; des weiteren können Nenbenwirkungen auftreten. Durch Derivatisierung gelingt es in einigen Fällen, diese Nachteile zu vermeiden.

Bereits bekannte derartige Derivate von Aminoglykosid- Antibiotika sind in 1-N-Stellung durch gegebenenfalls Substituenten aufweisende Acyl- oder Alkylreste substituierte Verbindungen wie 1-N-(4-Amino-2-hydroxybutyryl)-kanamycin A (FR—A 2 264 553), 1-N-Acetylsisomicin und 1-N-Äthylsisomicin (DT—OS 2 437 160 und DT—OS 2 552 799).

In der 1-Stellung Urethan- oder Harnstoffgruppen aufweisen- de Aminoglykoside sind hingegen bisher noch nicht bekannt geworden.

Es wurde nun gefunden, daß 1-N-4,6-Di-O-(aminoglykosyl)-1,3-diaminocyclitol-Derivate der Formel

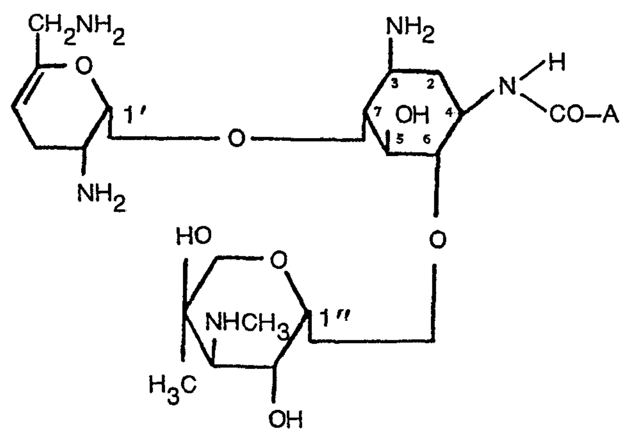

(I)

in der

A für OR oder

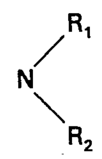

steht, wobei

R einen Alkylrest mit 1 bis 10 C-Atomen, der 1 oder 2 Substituenten aus der Reihe Halogen, Hydroxy, Mercapto, Cyan, Carboxy, Trifluormethyl, Alkoxy sowie Alkythio mit 1 bis 6 Kohlenstoffatomen, das im Alkylteil durch Amino, Monoalkyl- und Dialkylamino mit je 1 bis 4 C-Atomen je Alkylgruppe substituiert sein kann, Allyloxy, Phenoxy,

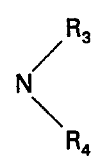

Alkoxycarbonyl mit 1 bis 4 C-Atomen im Alkylrest und Cycloalkyl mit 3 bis Kohlenstoffatomen im Cyclus tragen kann;

einen Alkenylrest mit 3 bis 7 C-Atomen;

einen Alkinylrest mit 3 oder 4 C-Atomen;

einen mono- oder bicyclischen Cycloalkylrest mit 3 bis 7 C-Atomen, der 1, 2 oder 3 Substituenten aus der Reihe Alkyl mit 1 bis 4 C-Atomen; Alkoxy mit 1 bis 4 C-Atomen; Hydroxy; Amino; Alkyl- und Dialkylamino mit 1 bis 4 Kohlenstoffatomen je Alkylgruppe aufweisen kann;

einen Phenyl- oder Benzylrest, der durch Nitro, Halogen oder Alkoxy mit 1 bis 4 C-Atomen substituiert sein kann;

einen Piperidinyl-, Tetrahydropyranyl-, Tetrahydrofuryl-, 1,3-Dioxolanyl- oder 1,3-Dioxolano [a,b] tetrahydrofurylrest, der durch 1 oder 2 Reste aus der Reihe Alkoxy mit 1 bis 4 C-Atomen; Hydroxy; Alkyl mit 1 bis 4 C-Atomen und 2,2-Dimethyl-1,3-dioxolanyl-4 substituiert sein kann, oder

einen 1,3-Dioxolanylalkyl-, Tetrahydrofurylalkyl-, Tetrahydropyranylalkyl-, Oxetanylalkyl-, 1,3-Oxathiolanylalkyl-, 1,3-Dithiolanylalkyl-, 1,4-Dioxaspiro[4,5]-decanyl-, Oxiranylalkyl-, Piperidinylalkyl-, Tetrahydropyrodinylalkyl- oder Aziridinylalkylrest, bei dem der Alkylrest 1 bis 4 Kohlenstoffatome enthält, und der 1 oder 2 Substituenten aus der Reihe Alkyl mit 1 bis 4 Kohlenstoffatomen; Phenyl und Alkoxy mit 1 bis 4 Kohlenstoffatomen tragen kann, darstellt,

$R_1$ die Bedeutung von Wasserstoff oder R hat,

$R_2$ für R, Wasserstoff oder eine 1-β-Tetra-O-acetyl-D-glucosyl-, 1-β-D-glucosyl-, Tetrahydropyridinyl-, Morpholino, Piperidino-, Alkoxy- mit 1 bis 4 C-Atomen, Cyclopentyloxy-, Cyclohexyloxy-, gegebenenfalls durch Halogen substituierte Benzyloxy-, Tetrahydropyranyloxy-, Tetrahydrofuranyloxy- oder Hydroxygruppe steht, oder

$R_1$—$R_2$ unter Einschluß des N-Atoms, an das sie gebunden sind, gemeinsam einen Pyrrolidino-, Piperidino-, Morpholino-, Piperazinyl-4-, Hexamethylenamino-, Isoxazolinyl-2- oder Tetrahydroisoxazinyl-2-rest darstellen, der 1 bis 2 Substituenten aus der Reihe Alkyl mit 1 bis 4 C-Atomen, das durch Hydroxy substituiert sein kann, tragen kann;

$R_3$ für Wasserstoff, $C_1$-$C_8$-Alkyl oder Allyl steht, und

$R_4$ Wasserstoff, $C_1$-$C_8$-Alkyl, $c_5$-$C_7$-Cycloalkyl, $C_5$-$C_7$-Cycloalkyl-$C_1$-$C_4$-alkyl, Phenyl, Phenyl-$C_1$-$C_4$-alkyl oder Allyl bedeutet und wobei die Alkyl-, Cycloalkyl- und Phenylreste $R_3$ und $R_4$ durch einen oder zwei Substituenten aus der Reihe Alkoxy mit 1 bis 4 C-Atomen, Amino, Monoalkyl- und Dialkylamino mit je 1 bis 4 C-Atomen je Alkylgruppe oder Hydroxy substituiert sein können,

sowie deren pharmazeutisch verwendbare Salze starke antibakterielle Eigenschaften gegen eine Vielzahl von Keimen und besonders gut Verträglichkeit besitzen.

Wie vergleichende Untersuchungen zeigten, haben diese erfindungsgemäßen neuen Aminoglykosid-Derivate erheblich stärkere antibiotische Wirkung als die Verbindungen des eingangs zitierten Standes der Technik.

Die pharmazeutisch verwendbaren Salze leiten sich von anorganischen oder organischen Säuren wie Schwefel-, Phosphor-, Salpeter-, Chlorwasserstoff-, Bromwasserstoff-, Essig-, Propion-, Ascorbin-, und Zitronensäure ab.

Als Alkyl in den Definitionel von R, $R_1$ und $R_2$ steht geradkettiges oder verzweigtes Alkyl mit 1 bis 10, insbesondere 1 bis 5 Kohlenstoffatomen. Beispielhaft seien Methyl, Athyl, n- und Isopropyl, n-, iso- und tert.-Butyl sowie Decyl genannt.

Als Alkenyl in den Definitionen von R, $R_1$ and $R_2$ steht geradkettiges oder verzweigtes Alkenyl mit vorzugsweise 3 bis 6 Kohlenstoffatomen. Beispielhaft seien genannt: Allyl, Isobutenyl, Dimethylallyl.

Als Alkinyl in den Definitionen von R, $R_1$ und $R_2$ steht geradkettiges oder verzweigtes Alkinyl mit 3 oder 4 Kohlenstoffatomen. Beispielhaft sei Propargyl genannt.

Als Cycloalkyl in den Definitionen von R, $R_1$ und $R_2$ steht mono- oder bicyclisches Cycloalkyl mit 3 bis 7 Kohlenstoffatomen. Beispielhaft seien Cyclopropyl, Cyclobutyl, Cyclopentyl, Cyclohexyl, Cycloheptyl und Bicyclo[2, 2, 1]-heptyl genannt.

Die oben genannten Alkyl-, Alkenyl-, Alkinyl-, Cycloalkyl-, Aryl-, Aralkyl-, Heterocyclyl- und Heterocyclylalkyl-Reste können durch einen oder mehrere, gleiche oder verschiedene Reste substituiert sein. Vorzugsweise enthalten sie 1 oder 2 Substituenten, von denen beispielhaft genannt seien:

Halogen, vorzugsweise Chlor oder Brom, Cyan, Hydroxy, Mercapto, Alkyl und Alkoxy mit 1 bis 6, insbesondere 1 bis 4 Kohlenstoffatomen, z.B. Methyl und Äthyl, Methoxy und Äthoxy; Allyloxy; Phenoxy, Carboxy, Carbamido, Alkoxycarbonyl mit 1 bis 4 Kohlenstoffatomen im Alkylteile, wie Athoxycarbonyl, Cycloalkyl mit 3 bis 6 Kohlenstoffatomen, z.B. Cyclopropyl oder Cyclohexyl, Amino, Alkyl- und Dialkylamino mit vorzugsweise 1 bis 6, insbesondere 1 bis 4 Kohlenstoffatomen je Alkylgruppe, die weiterhin durch Hydroxy, Alkoxy oder Amino substituiert sein kann, sowie Trifluormethyl.

Als neue Wirkstoffe seien im einzelnen genannt:

1-N-Methoxycarbonyl)-sisomicin, 1-N-Äthoxycarbonyl)-sisomicin, 1-N-Propoxycarbonyl)-sisomicin, 1-N-Isopropoxycarbonylsisomicin, 1-N-Butoxycarbonyl-sisomicin, 1-N-sek-Butoxycarbonylsisomicin, 1-N-tert.-Butoxycarbonyl-sisomicin, 1-N-Isobutoxycarbonyl-sisomicin, 1-N-Pentyloxycarbonyl-sisomicin, 1-N-(2,2-Dimethylpropoxycarbonyl)-sisomicin, 1-N-(3-Methylbutoxycarbonyl)-sisomicin,
1-N-Decyloxycarbonyl)-sisomicin,
1-N-(2-Chloräthoxycarbonyl)-sisomicin,
1-N-(4-Chlorbutoxycarbonyl)-sisomicin,
1-N-(2-Methoxyäthoxycarbonyl)-sisomicin,
1-N-(2,3-Dimethoxy-propoxycarbonyl)-sisomicin,
1-N-(2-Hydroxyäthoxycarbonyl)-sisomicin,
1-N-(2,2-Dimethoxyäthoxycarbonyl)-sisomicin,
1-N-(2-Dimethylaminoäthoxycarbonyl)-sisomicin,
1-N-(2-Cyclopentyloxycarbonyl)-sisomicin,
1-N-(2-Cyclohexyloxycarbonyl)-sisomicin,
1-N-(Cyclopropylmethoxycarbonyl)-sisomicin,

1-N-(Benzyloxycarbonyl)-sisomicin,
1-N-(Allyloxycarbonyl)-sisomicin,
1-N-(Propargyloxycarbonyl)-sisomicin,
1-N-(2-Phenoxyäthoxycarbonyl)-sisomicin,
1-N-(2,2-Dimethyl-1,3-dioxolan-4-yl-methoxycarbonyl)-sisomicin,
1-N-(Tetrahydrofuryl-2-methoxycarbonyl)-sisomicin,
1-N-(Phenoxycarbonyl)-sisomicin,
1-N-(4-Nitrophenoxycarbonyl)-sisomicin,
1-N-(1,2,5,6-Di-O-isopropyliden-$\alpha$-D-glucofuranosyl-3-carbonyl)-sisomicin,
1-N-(2,3-Dihydroxypropyloxycarbonyl)-sisomicin,
1-N-(1,3-Dihydroxy-2-propyloxycarbonyl)-sisomicin,
1-N-(3,4-Dihydroxybutyloxycarbonyl)-sisomicin,
1-N-(4,5-Dihydroxypentyloxycarbonyl)-sisomicin,
1-N-(5,6-Dihydroxyhexyloxycarbonyl)-sisomicin,
1-N-(2-Amino-3-hydroxy-propyloxycarbonyl)-sisomicin,
1-N-(3-Amino-2-hydroxy-propyloxycarbonyl)-sisomicin,
1-N-(2,3-Diaminopropyloxycarbonyl)-sisomicin,
1-N-(1,3-Diamino-2-propyloxycarbonyl)-sisomicin,
1-N-(2-Hydroxy-3-mercapto-propyloxycarbonyl)-sisomicin,
1-N-(2,3-Dimercapto-propyloxycarbonyl)-sisomicin,
1-N-(2-Amino-äthoxycarbonyl)-sisomicin,
1-N-(2-Amino-2-methyl-äthoxycarbonyl)-sisomicin,
1-N-(3-Amino-propoxycarbonyl)-sisomicin,
1-N-(2-Methylamino-äthoxycarbonyl)-sisomicin,
1-N-(1,3-Dioxolan-2-on-4-yl-methoxycarbonyl)-sisomicin,
1-N-(2-Methyl-1,3-dioxolan-4-yl-methoxycarbonyl)-sisomicin,
1-N-(2-Phenyl-1,3-dioxolan-4-yl-methoxycarbonyl)-sisomicin,
1-N-(2,2-Diäthyl-1,3-dioxolan-4-yl-methoxycarbonyl)-sisomicin,
1-N-(1,4-Dioxa-spiro[4,5]decan-2-yl-methoxycarbonyl)-sisomicin,
1-N-(2,2-Dimethyl-1,3-dioxolan-4-yl-2-äthoxycarbonyl)-sisomicin,
1-N-(2,2-Dimethyl-1,3-dioxolan-4-yl-3-propyloxycarbonyl)-sisomicin,
1-N-(2,2-Dimethyl-1,3-dioxolan-4-yl-4-butyloxycarbonyl)sisomicin,
1-N-(2,2-Dimethyl-1,3-oxathiolan-4-yl-methoxycarbonyl)-sisomicin,
1-N-(2,2-Dimethyl-1,3-dithiolan-4-yl-methoxycarbonyl)-sisomicin,
1-N-(2-Tetrahydropyranyl-methoxycarbonyl)-sisomicin,
1-N-(2-Methoxy-tetrahydropyran-3-yl-oxycarbonyl)-sisomicin,
1-N-(2-Methoxy-tetrahydrofuran-3-yl-oxycarbonyl)-sisomicin,
1-N-(2,5-Dimethoxy-tetrahydrofuran-3-yl-oxycarbonyl)-sisomicin,
1-N-(Oxiran-2-yl-methoxycarbonyl)-sisomicin,
1-N-(2-Äthyl-oxetan-2-yl-methoxycarbonyl)-sisomicin,
1-N-(2-Methoxycyclohexanyloxycarbonyl)-sisomicin,
1-N-Carbamoyl-sisomicin,
1-N-(Methylcarbamoyl)-sisomicin,
1-N-(Dimethylcarbamoyl)-sisomicin,
1-N-(Äthylcarbamoyl)-sisomicin,
1-N-(N-Äthyl-N-methylcarbamoyl)-sisomicin,
1-N-(Propylcarbamoyl)-sisomicin,
1-N-(N-Methyl-N-propylcarbamoyl)-sisomicin,
1-N-(Isopropylcarbamoyl)-sisomicin,
1-N-(Dipropylcarbamoyl)-sisomicin,
1-N-(Butylcarbamoyl)-sisomicin,
1-N-(tert.-Butylcarbamoyl)-sisomicin,
1-N-(Isobutylcarbamoyl)-sisomicin,
1-N-(sek.-Butylcarbamoyl)-sisomicin,
1-N-(Pentylcarbamoyl)-sisomicin,
1-N-(tert.-Pentylcarbamoyl)-sisomicin,
1-N-(Hexylcarbamoyl)-sisomicin,
1-N-(2-Äthylhexylcarbamoyl)-sisomicin,
1-N-(Decylcarbamoyl)-sisomicin,
1-N-(Methoxymethylcarbamoyl)-sisomicin,
1-N-(Äthoxymethylcarbamoyl)-sisomicin,
1-N-(Propoxymethylcarbamoyl)-sisomicin,
1-N-(Hexyloxymethylcarbamoyl)-sisomicin,
1-N-(2-Methoxyäthylcarbamoyl)-sisomicin,
1-N-(2-Chloräthylcarbamoyl)-sisomicin,

1-N-(Cyclohexylcarbamoyl)-sisomicin,
1-N-(Phenylcarbamoyl)-sisomicin,
1-N-(4-Methoxyphenylcarbamoyl)-sisomicin,
1-N-(Methoxycarbonylcarbamoyl)-sisomicin,
1-N-(Aethoxycarbonylcarbamoyl)-sisomicin,
1-N-(Phenoxycarbonylcarbamoyl)-sisomicin,
1-N-(Aethoxycarbonylmethylcarbamoyl)-sisomicin,
1-N-(1-β-D-Tetra-O-acetylglucoxylcarbamoyl)-sisomicin,
1-N-(1-β-D-Glucosylcarbamoyl)-sisomicin,
1-N-(2-Hydroxyathyl-methyl-carbamoyl)-sisomicin,
1-N-(2-Hydroxypropyl-carbamoyl)-sisomicin,
1-N-(Bis-[2-hydroxypropyl]-carbamoyl)-sisomicin,
1-N-(3-Hydroxybutyl-carbamoyl)-sisomicin,
1-N-(1,1-Dimethyl-2-hydroxy-äthylcarbamoyl)-sisomicin,
1-N-(1,1-Bis-[hydroxymethyl]-äthyl-carbamoyl)-sisomicin,
1-N-(1,1-Bis-[hydroxymethyl]-2-hydroxyäthyl-carbamoyl)-sisomicin,
1-N-(Allyl-carbamoyl)-sisomicin,
1-N-(Diallyl-carbamoyl)-sisomicin,
1-N-(Propargyl-carbamoyl)-sisomicin,
1-N-(2,2-Dimethoxyäthyl-carbamoyl)-sisomicin,
1-N-(2-Hydroxy-3-allyloxy-propyl-carbamoyl)-sisomicin,
1-N-(Carbamidomethyl-methyl-carbamoyl)-sisomicin,
1-N-(2-Dimethylaminoäthyl-carbamoyl)-sisomicin,
1-N-(2-Diathylaminoäthyl-carbamoyl)-sisomicin,
1-N-(3-Dimethylaminopropyl-carbamoyl)-sisomicin,
1-N-(3-Diäthylaminopropyl-carbamoyl)-sisomicin,
1-N-(1-Aethoxycarbonyläthyl-carbamoyl)-sisomicin,
1-N-(N-Cyclohexyl-N-methyl-carbamoyl)-sisomicin,
1-N-(N-Cyclohexyl-N-[2-hydroxyäthyl]-carbamoyl)-sisomicin,
1-N-(N-Cyclohexyl-N-[2-hydroxypropyl]-carbamoyl)-sisomicin,
1-N-(Cyclohexylmethyl-carbamoyl)-sisomicin,
1-N-(Pyrrolidino-carbonyl)-sisomicin,
1-N-(Piperidino-carbonyl)-sisomicin,
1-N-(Morpholino-carbonyl)-sisomicin,
1-N-(1-Methyl-4-piperazinyl-carbonyl)-sisomicin,
1-N-(1-[2-Hydroxyäthyl]-4-piperazinyl-carbonyl)-sisomicin,
1-N-(1-Hexamethylenamino-carbonyl)-sisomicin,
1-N-(Dimethylamino-carbamoyl)-sisomicin,
1-N-([N-Hydroxyäthyl-N-methylamino]-carbamoyl)-sisomicin,
1-N-(Morpholino-carbamoyl)-sisomicin,
1-N-(Hydroxy-carbamoyl)-sisomicin,
1-N-(M-Methoxy-carbamoyl)-sisomicin,
1-N-(N-Methoxy-N-methyl-carbamoyl)-sisomicin,
1-N-(Äthoxy-carbamoyl)-sisomicin,
1-N-(N-Äthoxy-N-athyl-carbamoyl)-sisomicin,
1-N-(Propoxy-carbamoyl)-sisomicin,
1-N-(N-Äthoxy-N-propyl-carbamoyl)-sisomicin,
1-N-(Butoxy-carbamoyl)-sisomicin,
1-N-(tert.-butoxy-carbamoyl)-sisomicin,
1-N-(Cyclohexyloxy-carbamoyl)-sisomicin,
1-N-(Benzyloxy-carbamoyl)-sisomicin,
1-N-(4-Chlorbenzyloxy-carbamoyl)-sisomicin,
1-N-(2,6-Dichlorbenzyloxy-carbamoyl)-sisomicin,
1-N-(2-Diäthylaminoäthoxy-carbamoyl)-sisomicin,
1-N-(2-Isoxazolinylcarbamoyl)-sisomicin,
1-N-(Tetrahydroisoxazinylcarbamoyl)-sisomicin,
1-N-(3-Cyanopropyl-carbamoyl)-sisomicin,
1-N-(3-Chlorpropyl-carbamoyl)-sisomicin,
1-N-(Cyanmethyl-carbamoyl)-sisomicin,
1-N-(2,2,2-Trifluoräthyl-carbamoyl)-sisomicin,
1-N-(N-Methyl-N-cyanmethyl-carbamoyl)-sisomicin,
1-N-(Bis-cyanmethyl-carbamoyl)-sisomicin,
1-N-(Aminocarbamoyl)-sisomicin,
1-N-(2,2-Diäthoxyäthyl-carbamoyl)-sisomicin,
1-N-(2-Hydroxyäthyloxycarbonylamino-carbamoyl)-sisomicin,

1-N-(D-Gluconamido-carbamoyl)-sisomicin,
1-N-(Methoxycarbonyl-carbamoyl)-sisomicin,
1-N-(Allyloxycarbonyl-carbamoyl)-sisomicin,
1-N-(1-Äthoxycarbonyläthyl-carbamoyl)-sisomicin,
1-N-(N-Methyl-N-morpholinocarbonyl-carbamoyl)-sisomicin,
1-N-(Allophanyl)-sisomicin,
1-N-(2-Äthoxycarbonylaminoäthyl-carbamoyl)-sisomicin,
1-N-(N-Methyl-N-aminocarbonylmethyl-carbamoyl)-sisomicin,
1-N-[4-(2-Hydroxyäthyl)-allophanyl]-sisomicin,
1-N-(2-Ureidoäthyl-carbamoyl)-sisomicin,
1-N-(2-Tetrahydropyranyloxy-carbamoyl)-sisomicin,
1-N-(2,2-Dimethyl-1,3-dioxolanyl-5-methyl-carbamoyl)-sisomicin,
1-N-(1,4-Dioxa-spiro[4,5]decan-2-yl-methyl-carbamoyl)-sisomicin,
1-N-(2-Tetrahydrofurylmethyl-carbamoyl)-sisomicin,
1-N-(2,3-Dihydroxypropyl-carbamoyl)-sisomicin,
1-N-(2-Hydroxyäthyl-carbamoyl)-sisomicin,
1-N-(2-Hydroxy-3-methoxypropyl-carbamoyl)-sisomicin,
1-N-(2-Aminoäthyl-carbamoyl)-sisomicin,
1-N-(2-Morpholinoäthyl-carbamoyl)-sisomicin,
1-N-(1-Methylpiperidinyl-4-methyl-carbamoyl)-sisomicin,
1-N-[3-(ziridinyl-1)-propyl-carbamoyl]-sisomicin,
1-N-(4-Piperidino-3-hydroxybutyl-carbamoyl)-sisomicin,
1-N-[3-Bis-(2-hydroxyäthyl)-aminopropyl-carbamoyl]-sisomicin,
1-N-[2-(N-2-Hydroxyäthyl-N-methylamino)-äthyl-carbamoyl]-sisomicin,
1-N-(2-Hydroxy-3-diäthylaminopropyl-carbamoyl)-sisomicin,
1-N-[3-(4-Methyl-1,2,5,6-tetrahydropyridinyl)-propyl-carbamoyl]-sisomicin,

Weitere erfindungsgemäße Wirkstoffe sind die im folgenden beispielhaft aufgezeigten 1-N-Aminoalkoxycarbonylderivate von Sisomicin, die also am 1-N-Atom eine Gruppe —CO—A tragen, wobei A für OR steht. Zur besseren Lesbarkeit und Übersicht wird lediglich der Substituent R wiedergegeben:

$—CH(CH_3)—CH_2—NH_2$

$—CH_2—C(CH_3)_2—NH_2$

$—CH(CH_3)—CH(CH_3)—NH_2$

$—C(CH_3)_2—CH_2NH_2$

$—CH_2—CH(CH_3)—NHCH_3$

$—CH(CH_3)—CH_2—NH—CH_3$

$—CH_2—CH_2—NH—C_2H_5$

$—CH—CH_2—NH_2$
$\quad|$
$\quad CH_2COOH$

$—CH—CH_2—NH_2$
$\quad|$
$\quad C_3H_7$

$—C(CH_3)_2—CH_2—NH—CH_3$

$—CH(CH_3)—CH(CH_3)—NHCH_3$

$—CH_2—C(CH_3)_2—NHCH_3$

$—CH_2—CH_2—NH—CH_2—CH=CH_2$

$—CH_2—CH_2—NH—C_3H_7$

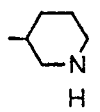 , 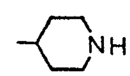

$—CH_2—CH_2—NH—(CH_2)_3—OC_2H_5$

$—CH_2—CH_2—NH—C(CH_3)_3$

$—CH_2—CH_2—NH—CH_2—CH(CH_3)_2$

$—CH(CH_3)—CH_2—NH—CH(CH_3)_2$

$—CH_2—CH_2—NH—(CH_2)_3—O—CH_3$

$—CH(CH_3)—CH_2—NH—(CH_2)_2—CH_3$

$—CH_2—CH_2—NH—C_4H_9$

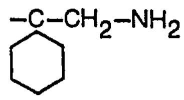 , 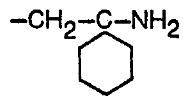

$—CH(CH_3)—CH_2—NH—C_4H_9$

$—CH(CH_3)—CH_2—NH—CH_2—CH(CH_3)_2$

$—CH_2—CH_2—CH(CH_3)—NH_2$

$—CH_2—CH(CH_3)—CH_2—NH_2$

$—CH_2—CH_2—CH_2—NH—CH_3$

$—CH_2—C(CH_3)_2—CH_2—NH_2$

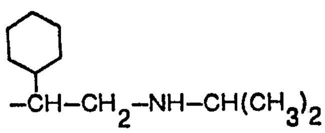 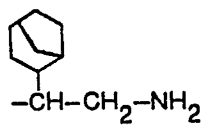

$—CH_2—CH_2—CH_2—NH—CH(CH_3)_2$

$—CH(CH_3)—CH_2—C(CH_3)_2—NH_2$

$—CH(CH_3)—CH_2—C(CH_3)_2—NHCH_3$

$—CH_2—CH_2—CH_2—NH—C_4H_9$

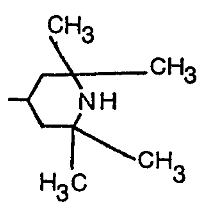

$—CH_2—CH_2—CH_2—CH_2—NH_2$

$—CH_2—CH_2—O—CH_2—CH_2—NH_2$

$—CH_2—CH_2—S—CH_2—CH_2—NH_2$

$—CH_2—CH_2—O—CH_2—CH_2—CH_2—NH_2$

$—CH_2—(CH_2)_4—NH_2$

$—CH_2—(CH_2)_2—CH(CH_3)—NH_2$

$—CH_2—(CH_2)_5—NH_2$

$-CH-CH_2-NH_2$
$|$
$CH_2NH_2$

$-CH_2-CH-CH_2-NH_2$
$|$
$NH_2$

$-CH_2-CH_2-NH-CH_2-CH_2-NH_2$

$-CH-CH_2-NH-CH_3$
$|$
$CH_2-NH-CH_3$

$-CH_2(CH_2)_2-NH-CH_2-CH_2-CH_2-NH_2$

$-CH_2-CH_2-NH-CH_2-C(CH_3)_2-NH_2$

$-CH-CH_2-NH-CH_2-NH_2$
$|$
$C_2H_5$

$-CH_2-CH_2-NH-CH_2-CH_2-CH_2-CH_2-NH_2$

$-CH_2-CH_2-NH-CH_2-CH_2-N(CH_3)_2$

$-CH(CH_3)-CH(CH_3)-NH-CH_2-CH_2-NH_2$

$-CH(CH_3)-CH_2-CH_2-NH-CH_2-CH_2-CH_2-NH_2$

$-CH_2-C(CH_3)_2-NH-CH_2-CH_2-CH_3$

$-CH-CH_2-NH_2$
$|$
$CH_2-N(C_2H_5)_2$

$-CH(CH_3)-CH_2-NH-CH_2-(CH_2)_2-NH_2$

$-CH_2-CH_2-NH-CH_2-CH_2-CH_2-NH-C(CH_3)_3$

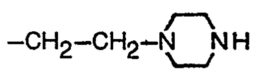

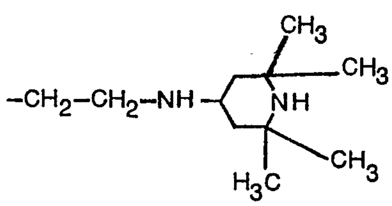

$-CH_2-CH_2-NH-CH_2-CH_2-NH-CH_2-CH_2-NH_2$

$-CH_2-CH-CH_2-NH_2$
$|$
$OH$

$-CH_2-CH-OCOCH_3$
$|$
$NH_2$

Es wurde weiterhin gefunden, daß man die erfindungsgemäßen Sisomicin-Derivate erhält, wenn man Verbindungen der Formel

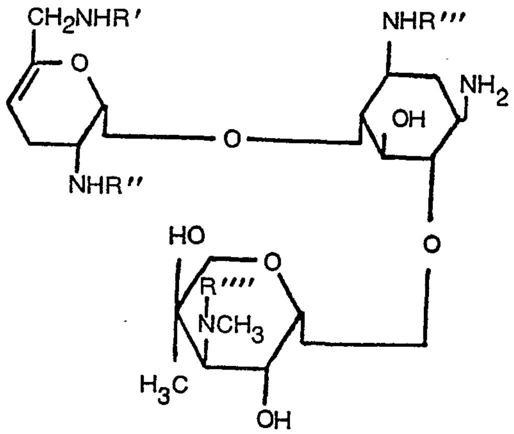

(II)

worin
R′, R″, R‴, und R″″ Wasserstoff oder leicht abspaltbare Aminoschutzgruppen bedeuten oder ihre Salze. die einen anorganischen oder organischen Säurerest enthalten, in an sich bekannter Weise mit einem Acylierungsmittel der Formel

A′—CO—E (III)

in welcher
A′ die oben für A angegebene Bedeutung besitzt, mit der Ausnahme, daß in A gegebenenfalls vorhandene freie Aminogruppen mit den üblichen Aminoschutzgruppen wie sie z.B aus der Literaturstelle Houben-Weyl, Methoden der Organischen Chemie, Band XV/1, Seiten 46—305, Georg Thieme Verlag, Stuttgart, 1974 bekannt sind, vorzugsweise jedoch die o-Nitrophenylsulfenyl-Schutzgruppen, blockiert sind und
E für Halogen, vorzugsweise CL oder Br, $N_3$, —O—CO—O—A′, Phenoxy, 4-Nitrophenoxy, 2,4,5-Trichlorphenoxy oder

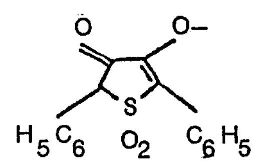

steht, umsetzt und den Ansatz in üblicher Weise, gegebenenfalls unter Abspaltung der Schutzgruppen, auf Verbindungen der Formel I hin aufarbeitet bzw. diese in ihre pharmazeutisch verwendbaren Salze überführt.

Darüber hinaus erhält man Verbindungen der Formel (I), in der A die Bedeutung von

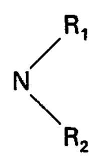 besitzt,

wenn man eine Verbindung der Formel

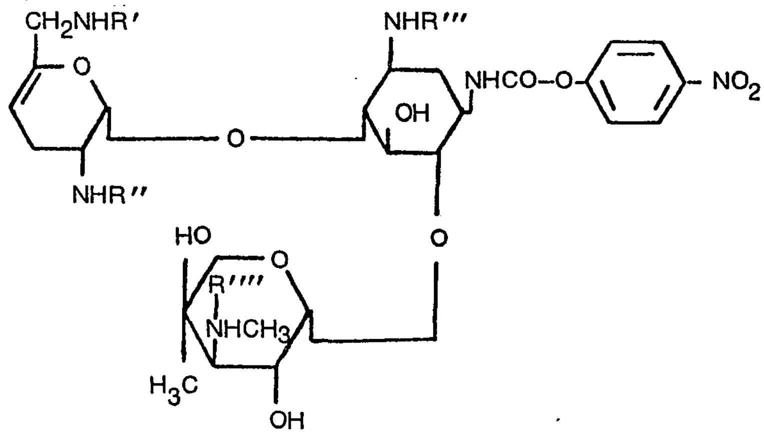

(IV)

worin
R', R'', R''' und R'''' die oben angegebene Bedeutung haben, mit Verbindungen der Formel

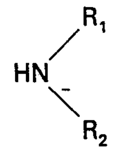

(V)

worin
$R_1$ und $R_2$ obengenannte Bedeutung haben, umsetzt, die Schutzgruppen abspaltet und die gewünschten Produkte isoliert.

Des weiteren erhält man Verbindungen gemäß Formel (I), in welcher A eine Gruppe NH—$R_2$ bedeutet, wenn man Verbindungen der Formel (II)
mit einem Isocyanat der Formel

$$R_2—NCO \qquad (VI)$$

wobei $R_2$ die oben angegebene Bedeutung besitzt, umsetzt, die Schutzgruppen abspaltet und gegebenenfalls die 1-N-4,6-Di-O-(aminoglykosyl)-1,3-diamino-cyclitol-Derivate durch Umsetzung mit geeigneten Säuren in ihre pharmazeutisch verwendbaren Salze überführt.

Unter leicht abspaltbaren Schutzgruppen sind solche zu verstehen, die bei Peptid-Synthesen zum Schutz der Aminogruppen Verwendung finden und sich durch schonende Hydrolyse, schonende Hydrogenolyse oder nucleophile Verdrängungsreaktionen unter Erhalt der eingeführten 1-N-Acylgruppe und ohne Beeinflussung des 4,6-Di-O-(aminoglykosyl)-1,3-diaminocyclitol-Gerüstes wieder entfernen lassen. Es sind mehrere solcher Schutzgruppen bekannt (Houben-Weyl, Methoden der Organischen Chemie, Band XV/1, Seiten 46—305, Georg-Thieme Verlag, Stuttgart 1974).

Gemäß Formel II kann eines oder es können bis auf 1-N alle N-Atome blockiert sein; weiterhin ist es möglich, von den freien 4,6-Di-O-(aminoglykosyl)-1,3-diaminocyclitolen bzw. von deren Säureadditionssalzen auszugehen.

Als N-Schutzgruppen R', R'', R''' und R'''' seien beispielhaft die folgenden Reste genannt: o-Nitrophenylsulfenyl, 2,4-Dinitrophenylsulfenyl, Pentachlorphenylsulfenyl, Tritylsulfenyl, Carbobenzoxy, Phthaloyl, tert-Butoxycarbonyl und Trifluoracetyl.

Verwendet man 2', 3, 3'', 6' - Tetra - N - (o - nitrophenylsulfenyl) - sisomicin und 4 - (2,2 - Dimethyl - 1,3 - dioxolan - 4 - yl - methoxycarbonyloxy) - 3 - oxo - 2,5 - diphenyl - 2,3 - dihydrothiophen - 1,1 - dioxid als Ausgangsstoffe, so kann der Reaktionsablauf durch das folgende Formelschema wiedergegeben werden:

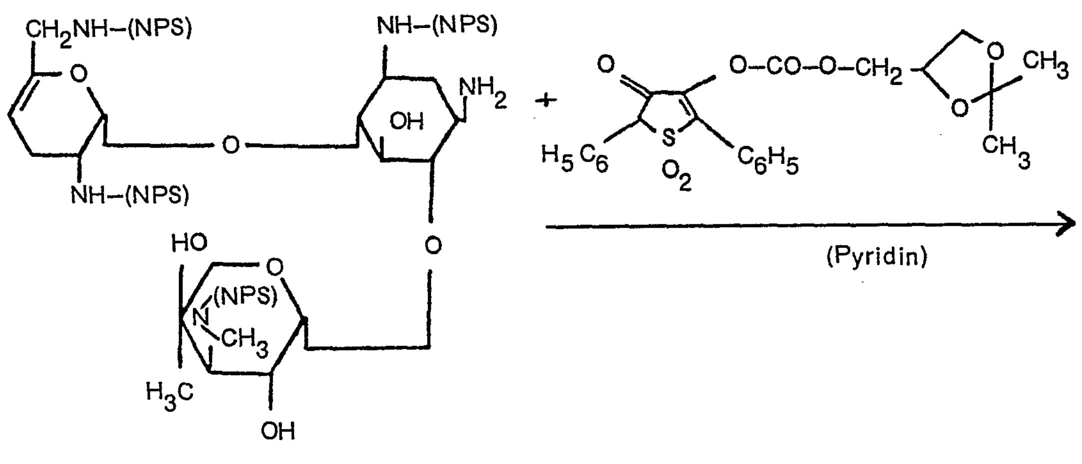

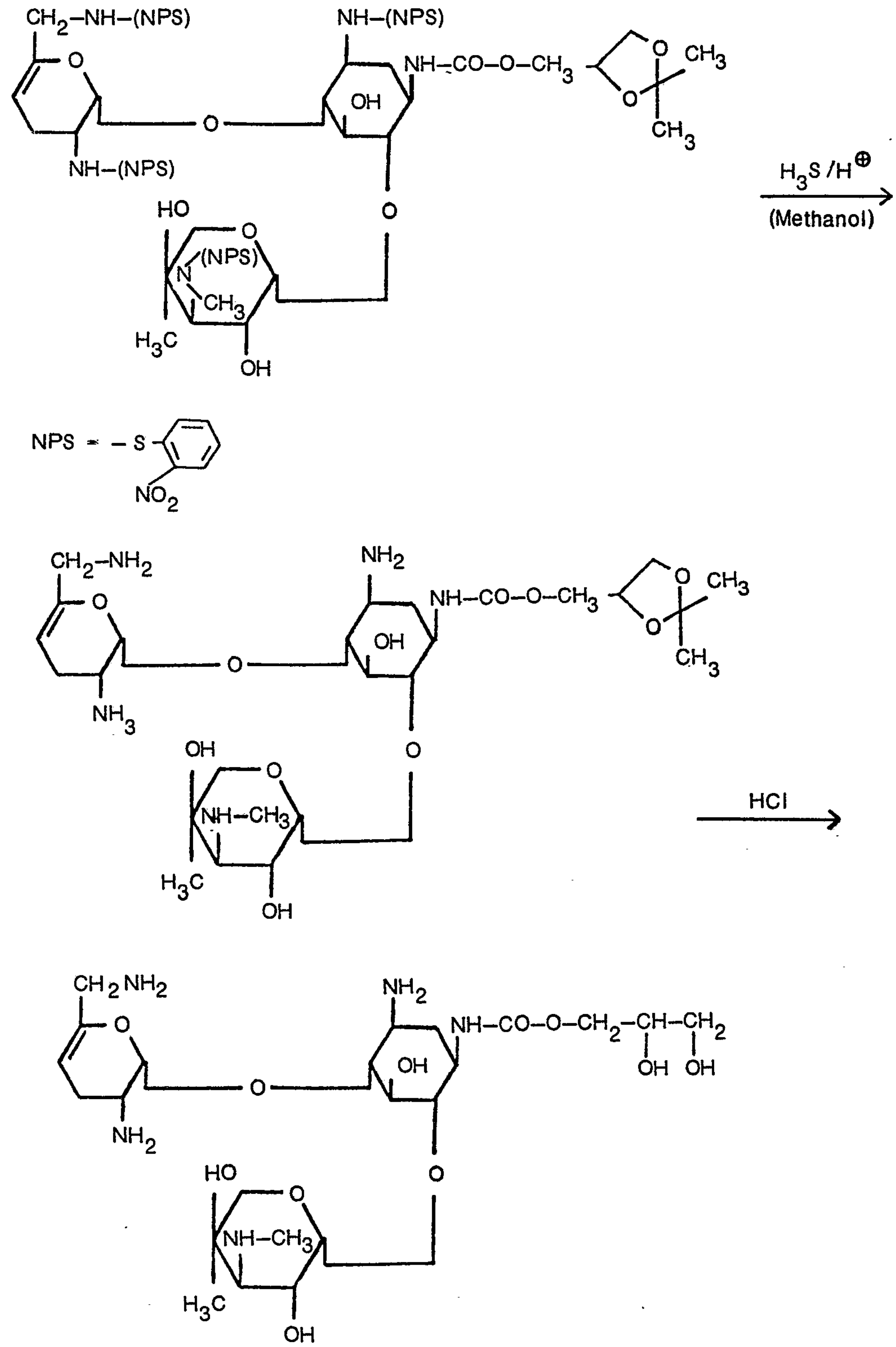
H3S /H⊕
(Methanol)
HCl

Verwendet man 2', 3, 3", 6'-Tetra-N-(o-nitrophenylsulfenyl)-1-N-(p-nitrophenoxycarbonyl)-sisomicin und Hydroxylamin als Ausgangsstoffe, so kann der Reaktionsablauf durch das folgende Formelschema wiedergegeben werden:

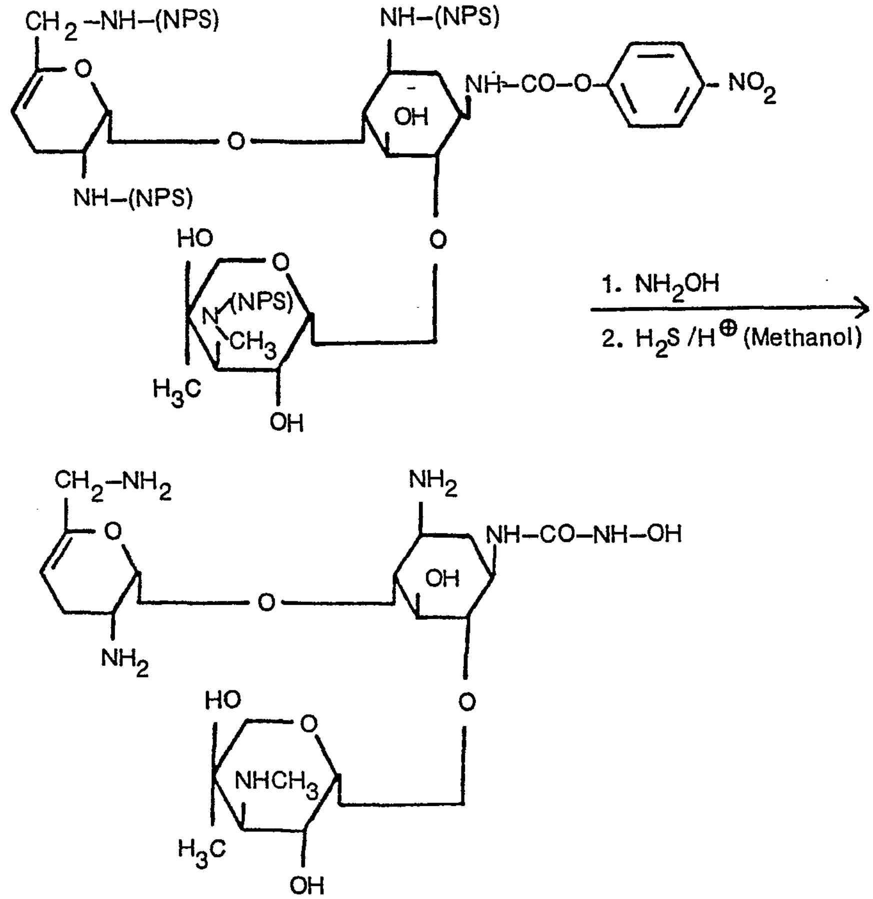


Bevorzugt werden in das Verfahren zur Herstellung der erfindungsgemäßen 1-N-4,6-Di-O-(aminoglykosyl)-1,3-diaminocyclitol-Derivate solche N-geschützten Derivate gemäß den Formeln II und IV eingesetzt, bei denen

R', R", R"' und R"" für Sulfenylschutzgruppen vom Typus —$SR_5$ stehen, wobei $R_5$ Phenyl, substituiertes Phenyl, Di- oder Triphenylmethyl bedeutet.

Zur Herstellung der solcherart geschützten 4,6-Di-O-(aminoglykosyl)-1,3-diaminocyclitole setzt man nach einem neuen Verfahren (Europäische Patentanmeldung 78100099.7) Sisomicin mit etwa 3 bis etwa 4 Äquivalenten einer Verbindung der Formel VII

$$R_5—S—G \qquad (VII)$$

in der $R_5$ die oben angegebene Bedeutung besitzt und G Halogen oder eine bei Sulfenylierungsreaktionen gebräuchliche Abgangsgruppe bezeichnet, in einem inerten Lösungsmittel bei Temperaturen zwischen etwa −30°C und +50°C in Gegenwart einer Base um und arbeitet das Reaktionsprodukt in üblicher Weise auf.

Als Beispiele für solcherart geschützte Verbindungen seien genannt:
2',3,3",6'-Tetra-N-(o-nitrophenylsulfenyl)-sisomicin,
2',3,3",6'-Tetra-N-(2,4-dinitrophenylsulfenyl)-sisomicin,
2',3,3",6'-Tetra-N-(pentachlorphenylsulfenyl)-sisomicin,
2',3,3",6'-Tetra-N-(tritylsulfenyl)-sisomicin,
2',3,3",6'-Tetra-N-(butoxycarbonyl)-sisomicin,
2',3,3",6'-Tetra-N-(trifluoracetyl)-sisomicin.

Die als Acylierungsmittel verwendeten Ausgangsstoffe der Formeln III und VI sind bekannt oder können nach prinzipiell bekannten Verfahren erhalten werden. So lassen sich die Verwendeten 4-Acyloxy-3-oxo-2,5-diphenyl-2,3-dihydrothiophen-1,1-dioxide beispielsweise durch Erwärmen von 4,6-Diphenylthieno [3,4-d] [1,3]dioxol-2-on-5,5-dioxid (vgl. Angew. Chem. *88*, 480 (1976)) mit Alkoholen oder Aminen in inerten Lösungsmitteln wie Toluol oder Acetonitril gewinnen.

Die eingesetzten (o-Nitrophenylsulfenyl-aminoalkyl-)-(p-nitrophenyl)-carbonate werden durch

Umsetzung der (o-Nitrophenylsulfenyl)-aminoalkohole mit Chlorkohlensäure-p-nitrophenylester in Gegenwart eines Säurebinders wie z.B. Triäthylamin in inerten Verdunnungsmitteln wie Dichlormethan oder Acetonitril erhalten.

Als Beispiele für Acylierungsmittel seien genannt: Chlorkohlensäure-methylester, Chlorkohlensäure-äthylester, Chlorkohlensäure-propylester, Chlorkohlensäure-isopropylester, Chlorkohlensäure-cyclohexylester, Chlorkohlensäure-2,2,2-trichloräthylester, Chlorkohlensäure-2-bromäthylester, Chlorkohlensäure-4-nitrophenylester, tert-Butoxycarbonylazid, Pyrokohlensauredimethylester, Pyrokohlensäurediäthylester, Pyrokohlensaure-ditert-butylester, Methoxycarbamidsäurephonylester, N-Methoxy-N-methyl-carbamidsäure-2,4,5-trichlorphenylester, Dimethylcarbonidsäurechlorid,
4-Diathylaminocarbonyloxy-3-oxo-2,5-diphenyl-2,3-dihydrothiophen-1,1-dioxid,
4-Benzylaminocarbonyloxy-3-oxo-2,5-diphenyl-2,3-dihydrothiophen-1,1-dioxid,
Methylisocyanat, Aethylisocyanat, Propylisocyanat, Isopropylisocyanat, Butylisocyanat, Isobutylisocyanat, Pentylisocyanat, Hexylisocyanat, Methoxymethylisocyanat, Aethoxymethylisocyanat, Propoxymethylisocyanat, Hexyloxymethylisocyanat, 2-Methyloxyäthylisocyanat, 2-Chloräthylisocyanat, Cyclohexylisocyanat, Phenylisocyanat, 4-Methoxyphenylisocyanat, Methoxycarbonylisocyanat, Aethoxycarbonylisocyanat, Phenoxycarbonylisocyanat, Isocyanatoessigsäureäthylester, 1-$\beta$-Isocyanato-tetra-O-acetyl-D-glucose.

Als Beispiele neuer Acylierungsmittel seien genannt:
4-Diäthylaminocarbonyloxy-3-oxo-2,5-diphenyl-2,3-dihydrothiophen-1,1-dioxid,
4-Benzylaminocarbonyloxy-3-oxo-2,5-diphenyl-2,3-dihydrothiophen-1,1-dioxid,
4-Methoxycarbonyloxy-3-oxo-2,5-diphenyl-2,3-dihydrothiophen-1,1-dioxid,
4-Äthoxycarbonyloxy-3-oxo-2,5-diphenyl-2,3-dihydrothiophen-1,1-dioxid,
4-Propoxycarbonyloxy-3-oxo-2,5-diphenyl-2,3-dihydrothiophen-1,1-dioxid,
4-Isopropoxycarbonyloxy-3-oxo-2,5-diphenyl-2,3-dihydrothiophen-1,1-dioxid,
4-Butoxycarbonyloxy-3-oxo-2,5-diphenyl-2,3-dihydrothiophen-1,1-dioxid,
4-Cyclohexyloxycarbonyloxy-3-oxo-2,5-diphenyl-2,3-dihydrothiophen-1,1-dioxid,
4-Cyclopropylmethoxycarbonyloxy-3-oxo-2,5-diphenyl-2,3-dihydrothiophen-1,1-dioxid,
4-Benzyloxycarbonyloxy-3-oxo-2,5-diphenyl-2,3-dihydrothiophen-1,1-dioxid,
4-Allyloxycarbonyloxy-3-oxo-2,5-diphenyl-2,3-dihydrothiophen-1,1-dioxid,
4-Propargyloxycarbonyloxy-3-oxo-2,5-diphenyl-2,3-dihydrothiophen-1,1-dioxid,
4-(2-Phenoxyäthoxycarbonyloxy-3-oxo-2,5-diphenyl-2,3-dihydrothiophen-1,1-dioxid,
4-(2,2-Dimethyl-1,3-dioxolanyl-4-methoxycarbonyloxy)-3-oxo-2,5-diphenyl-2,3-dihydrothiophen-1,1-dioxid,
4-(Tetrahydrofuryl-2-methoxycarbonyloxy-3-oxo-2,5-diphenyl-2,3-dihydrothiophen-1,1-dioxid,
4-(Tetrahydropyranyl-2-methoxycarbonyloxy-3-oxo-2,5-diphenyl-2,3-dihydrothiophen-1,1-dioxid,
4-(1,2,5,6-Di-O-isopropyliden-$\alpha$,D-glucofuranosyl-3-carbonyloxy)-3-oxo-2,5-diphenyl-2,3-dihydrothiophen-1,1-dioxid,
4-Isobutoxycarbonyloxy-3-oxo-2,5-diphenyl-2,3-dihydrothiophen-1,1-dioxid,
4-tert.-Butoxycarbonyloxy-3-oxo-2,5-diphenyl-2,3-dihydrothiophen-1,1-dioxid,
4-sek-Butoxycarbonyloxy-3-oxo-2,5-diphenyl-2,3-dihydrothiophen-1,1-dioxid,
4-(2-Chloräthoxycarbonyloxy-3-oxo-2,5-diphenyl-2,3-dihydrothiophen-1,1-dioxid,
4-(4-Chlorbutoxycarbonyloxy-3-oxo-2,5-diphenyl-2,3-dihydrothiophen-1,1-dioxid,
4-Pentyloxycarbonyloxy-3-oxo-2,5-diphenyl-2,3-dihydrothiophen-1,1-dioxid,
4-(3-Methyl-butoxycarbonyloxy-3-oxo-2,5-diphenyl-2,3-dihydrothiophen-1,1-dioxid,
4-(2,2-Dimethyl-propoxycarbonyloxy-3-oxo-2,5-diphenyl-2,3-dihydrothiophen-1,1-dioxid,
4-Heptyloxycarbonyloxy-3-oxo-2,5-diphenyl-2,3-dihydrothiophen-1,1-dioxid,
4-Decyloxycarbonyloxy-3-oxo-2,5-diphenyl-2,3-dihydrothiophen-1,1-dioxid,
4-(2-Methoxy-äthoxycarbonyloxy)-3-oxo-2,5-diphenyl-2,4-dihydrothiophen-1,1-dioxid,
4-(2,3-Dimethoxy-propoxycarbonyloxy-3-oxo-2,5-diphenyl-2,3-dihydrothiophen-1,1-dioxid,
4-(2-Hydroxy-äthoxycarbonyloxy)-3-oxo-2,5-diphenyl-2,3-dihydrothiophen-1,1-dioxid,
4-(2,2-Dimethoxy-äthoxycarbonyloxy)-3-oxo-2,5-diphenyl-2,3-dihydrothiophen-1,1-dioxid,
4-(2-Dimethylaminoäthoxycarbonyloxy)-3-oxo-2,5-diphenyl-2,3-dihydrothiophen-1,1-dioxid,
4-Cyclopentyloxycarbonyloxy-3-oxo-2,5-diphenyl-2,3-dihydrothiophen-1,1-dioxid,

Neue Acylierungsmittel der Formel III, bei denen vorhandene Aminofunktionen durch die o-Nitrophenylsulfenyl (NPS)-Schutzgruppe blockiert sind, werden im folgenden aufgeführt. Diese neuen Verbindungen entsprechen der Formel

$$A'—CO—E,$$

wobei E für p-Nitrophenoxy steht. A' kann beispielsweise folgende Bedeutung annehmen:

$O-CH_2-CH_2-NH-NPS$

$O-CH_2-CH(CH_3)-NH-NPS$

$O-CH(CH_3)-CH_2-NH-NPS$

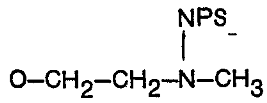

$O-CH_2-C(CH_3)_2-NH-NPS$

$O-CH(CH_3)-CH(CH_3)-NH-NPS$

$O-C(CH_3)_2-CH_2-NH-NPS$

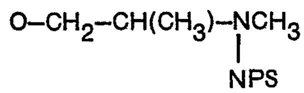

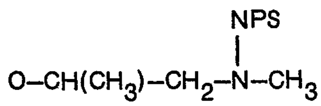

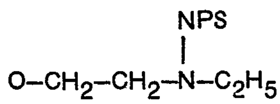

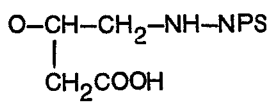

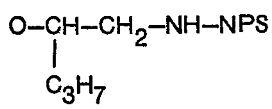

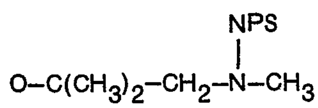

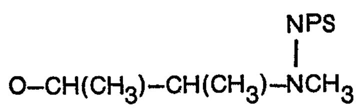

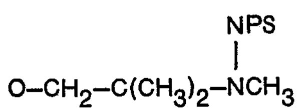

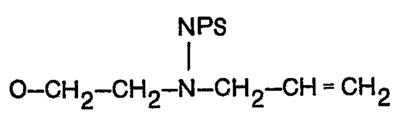

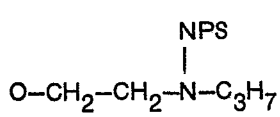

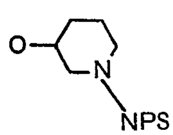 , 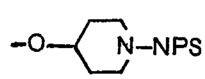

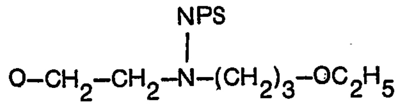

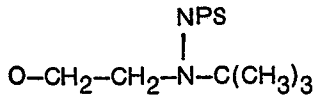

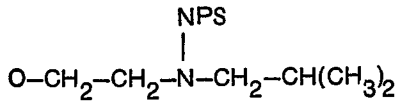

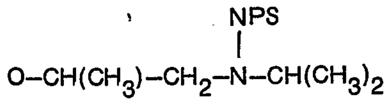

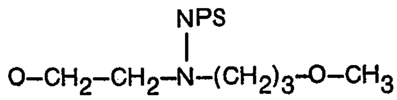

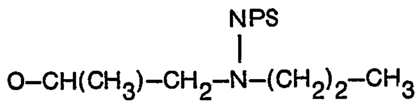

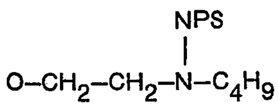

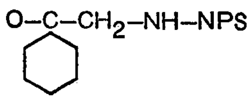

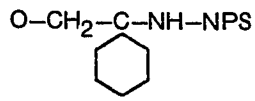

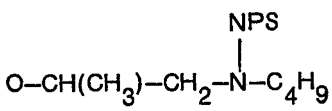

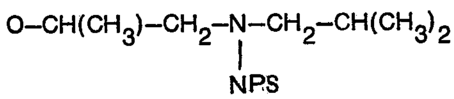

$O-CH_2-CH_2-CH_2-NH-NPS$

$O-CH_2-CH_2-CH(CH_3)-NH-NPS$

$O-CH_2-CH(CH_3)-CH_2-NH-NPS$

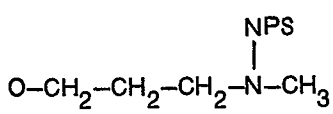

$O-CH_2-C(CH_3)_2-CH_2-NH-NPS$

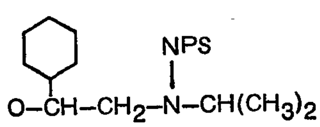 , 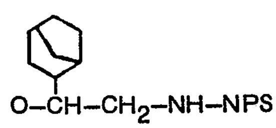

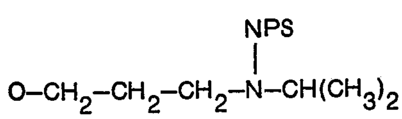

$O-CH(CH_3)-CH_2-C(CH_3)_2-NH-NPS$

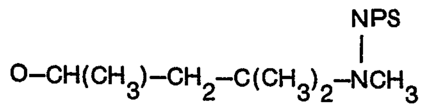

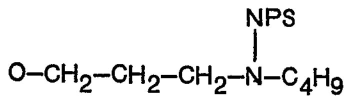

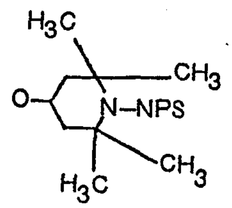

$O-CH_2-CH_2-CH_2-CH_2-NH-NPS$

$O-CH_2-CH_2-O-CH_2-CH_2-NH-NPS$

$O-CH_2-CH_2-S-CH_2-CH_2-NH-NPS$

$O-CH_2-CH_2-O-CH_2-CH_2-CH_2-NH-NPS$

$O-CH_2-(CH_2)_4-NH-NPS$

$O-CH_2-(CH_2)_2-CH(CH_3)-NH-NPS$

$O-CH_2-(CH_2)_5-NH-NPS$

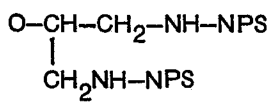

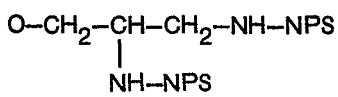

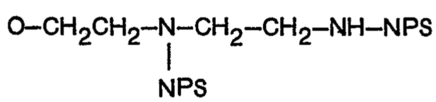

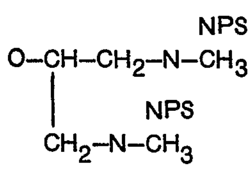

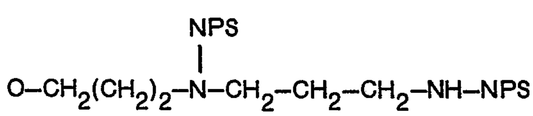

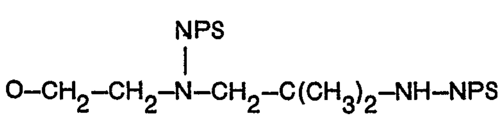

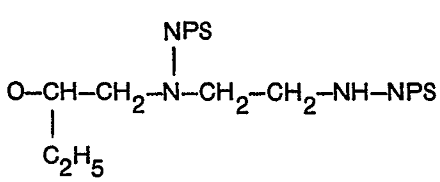

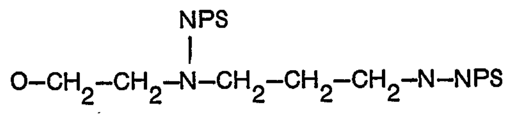

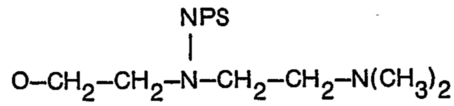

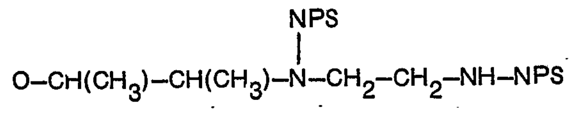

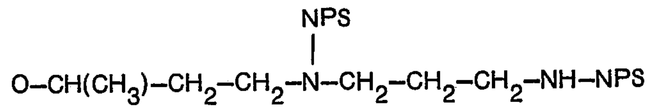

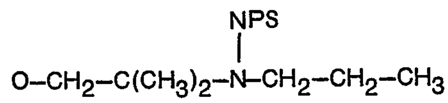

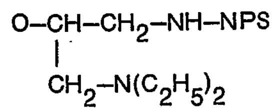

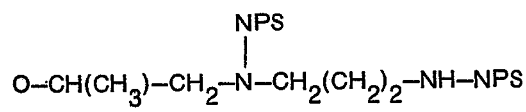

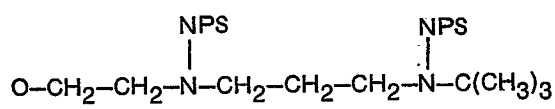

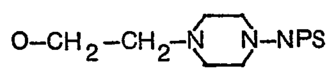

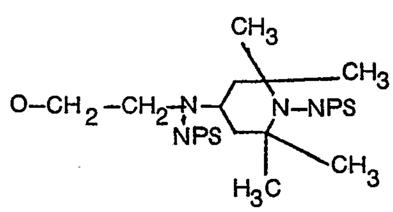

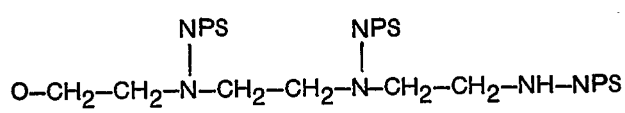

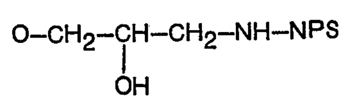

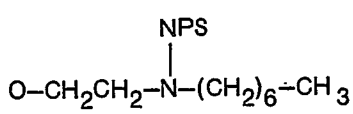

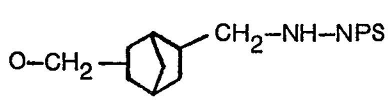

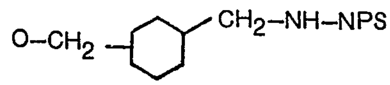

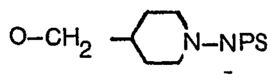

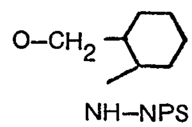

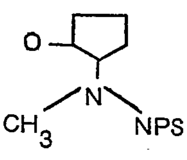

Die als Zwischenprodukte verwendeten Verbindungen der Formel IV sind ebenfalls neu und werden durch Acylierung von Verbindungen der Formel II mit Chlorameisensäure-p-nitrophenylester nach dem in dieser Anmeldung beschriebenen Verfahren hergestellt.

Die Aminoverbindungen der Formel V sind bekannt. Als Beispiele seien genannt:

Ammoniak, Methylamin, Diamethylamin, Äthylamin, Äthylmethylamin, Diäthylamin, Propylamin, Methylpropylamin, Dipropylamin, Butylamin, Isobutylamin, tert.-Butylamin, sek.-Butylamin, Pentylamin, tert.-Pentylamin, 2-Äthylhexylamin, Decylamin, 2-Hydroxyäthylamin, 2-Methylaminoäthanol, 1-Amino -propanol, Bis-(2-hydroxypropyl)-amin, 4-Amino-2-butanol, 2-Amino-2-methyl-propanol, 2-Amino-2-methyl-1,3-propandiol, 2-Amino-1-hydroxymethyl-1,3-propandiol, 2-Methoxyäthylamin, 3-Chlorpropylamin, 3-Cyanopropylamin, 3-Methylaminopropannitril, Allylamin, Diallylamin, Propargylamin, 2,2-Dimethoxyäthylamin, 2-Hydroxy-4-allyloxypropylamin, Methylaminoacetamid, 1-Amino-2-dimethylaminoäthan, 1-Amino-2-diäthylaminoäthan, 1-Amino-3-dimethylaminopropan, 1-Amino-3-diäthylaminopropan, Alaninäthylester, Cyclohexylamin, N-Methylcyclohexylamin, N-(2-Hydroxyäthyl)-cyclohexylamin, 1-(Cyclohexylamino)-2-propanol, Aminomethylcyclohexan, Pyrrolidin, Piperidin, Morpholin, 1-Methylpiperazin, 1-(2-Hydroxyäthyl)-piperazin, Hexamethylenamin, N,N-Dimethylhydrazin, N-Hydroxyäthyl-N-methyl-hydrazin, 4-Aminomorpholin, Hydroxylamin, Methoxyamin, O,N-Dimethylhydroxylamin, Äthoxyamin, O,N-Diäthylhydroxylamin, Propoxamin, O-Äthyl-N-propyl-hydroxylamin, Butoxyamin, tert.-Butoxyamin, Cyclohexyloxyamin, Benzyloxyamin, 4-Chlorbenzyloxyamin, 2,6-Dichlorbenzyloxyamin, O-(2-Diäthylaminoäthyl)-hydroxylamin, Isoxazolidin, Tetrahydroisoxazin.

Als Verdünnungsmittel kommen alle inerten organischen Lösungsmittel in Frage. Hierzu gehören vorzugsweise Toluol, Chloroform, Methylenchlorid, Dimethylformamid, Dimethylacetamid, Dimethylsulfoxid, Äther wie Diäthyläther, Dioxan und Tetrahydrofuran, Pyridin, Alkohole wie Methanol und Äthanol sowie deren Gemische.

Werden Säurebinder benötigt, so können alle üblichen organischen und anorganischen Säurebindungsmittel verwendet werden. Hierzu gehören vorzugsweise Alkali- und Erdalkalihydroxide wie Natriumhydroxid, Kaliumhydroxid oder Calciumhydroxid, Alkali- und Erdalkalicarbonate und -hydrogencarbonate wie Natriumcarbonat, Kaliumcarbonat, Natriumhydrogencarbonat und Calciumcarbonat, Calciumoxid, tertiäre aliphatische und aromatische Amine wie Triäthylamin und N,N Dimethylanilin sowie heterocyclische Basen wie Pyridin und Chinolin.

Die Reaktionstemperaturen können in einem großen Bereich variiert werden. Im allgemeinen arbeitet man bei Temperaturen von etwa —30°C bis +80°C, vorzugsweise zwischen etwa 0°C und etwa +25°C.

Die Umsetzung kann bei Normaldruck, aber auch bei erhöhtem Druck durchgeführt werden. Im allgemeinen arbeitet man bei Normaldruck.

Bei der Durchführung des erfindungsgemäßen Verfahrens setzt man 1 Mol der Verbindung der Formel II mit etwa 1 bis 3 Mol, vorzugsweise mit 1,1 bis 1,5 Mol einer Verbindung der Formel III oder VI oder 1 Mol der Verbindung der Formel IV mit etwa 1 bis 3 Mol, vorzugsweise 1,1 bis 1,5 Mol der Verbindung der Formel V um. Man arbeitet bevorzugt in Pyridin als Verdünnungsmittel bei Raumtemperatur. Anschließend werden die Schutzgruppen abgespalten und in üblicher Weise auf das freie 1-N-Derivat hin aufgearbeitet und dieses gegebenenfalls in die pharmazeutisch verwendbaren Salze überführt. Verwendet man beispielsweise die besonders bevorzugten o-Nitrophenylsulfenyl-Schutzgruppen, so erfolgt deren schonende Abspaltung mit schwefelhaltigen, nucleophilen Reagentien wie $H_2S$ oder Thiophenol.

Die erfindungsgemäßen Verbindungen sind antimikrobielle Mittel mit einem breiten Wir-

kungsspektrum und besonderer Wirksamkeit gegen gram-negative Bakterien. Diese Eigenschaften ermöglichen ihre Verwendung als Arzneimittel, insbesondere bei der Bekämpfung von durch Bakterien hervorgerufenen Erkrankungen bei Mensch und Tier. Sie sind gut zur Prophylaxe und Chemotherapie von lokalen und systemischen Infektionen, insbesondere Infektionen des Urogenitalsystems in der Human- und Tiermedizin geeignet, die durch gram-negative Bakterien, z.B. E.coli, Proteus, Klebsiella und Pseudomonas verursacht werden, geeignet, Hemmhöfe im Agar-Lochtest wurden z.B. gegen folgende Bakterienstämme bei einer Konzentration von 100 Mikrogramm/1 ml gefunden:

| | |
|---|---|
| Pseudomonas aerug. | 5737 |
| Pseudomonas aerug. | F 41 |
| Klebsiella pneum. | 2 München |
| Klebsiella pneum. | 1 Düsseldorf |
| E. coli | Münster |
| E. coli | Neumann |

Das Verfahren zur Behandlung von durch Bakterien hervorgerufenen Erkrankungen ist dadurch gekennzeichnet, daß man die neuen 1-N-Derivate Menschen und Tieren appliziert, die an diesen Erkrankungen leiden.

Im allgemeinen hängt die zu verabreichende Dosis der Verbindungen der Erfindung vom Alter und Gewicht des Lebewesens, von der Verabreichungsart, vom Typ und von der Schwere der bakteriellen Infektion ab. Die Dosierung der erfindungagemäßen Verbindungen ist gewöhnlich der Dosierung der 1-N-unsubstituierten Verbindungen ähnlich. Der Dosierungsspielraum beträgt von 20 mg/Tag/Mensch bis 2000/mg/Tag/Mensch, vorzugsweise 100 mg—500 mg/Tag.

Die Verbindungen der Erfindung können auch topisch verabreicht werden in der Form von Salben, Cremen oder Lotionen. Pharmazeutische Trägerstoffe für diese Formulierungen umfassen Wasser, Öle, Fette, Polyester und Polyole.

Wie im folgenden gezeigt wird ist das Verfahren zur Herstellung von Arzneimitteln, welche die neuen 1-N-Sisomicin-Derivate enthalten, dadurch gekennzeichnet, daß man die neuen Verbindungen mit pharmazeutisch geeigneten Träger und/oder Zusatzstoffen vermischt.

Im allgemeinen enthalten topische Präparationen ca. 0,1 bis ca. 3,0 g der Verbindungen der Erfindung pro 100 g Salbe, Creme oder Lotion. Die topische Verabreichung erfolgt ca. 2 bis 5 mal am Tag.

Die antibakteriellen Mittel der Erfindung können in flüssiger Form als Lösungen oder Suspensionen zur Anwendung an Ohren und Augen oder zur parenteralen Verabreichung in Form intramuskulärer Injektionen vorliegen. Injektionslösungen oder -suspensionen werden gewöhnlich so verabreicht, daß ca. 1 bis 15 mg Wirkstoff pro Kilogramm Körpergewicht in 2 bis 4 Dosen pro Tag in den infizierten Organismus gelangen. Die genaue Dosis hängt von der Art der Infektionen, der Empfindlichkeit des infizierenden Keims und von den individuellen Charakteristika des zu Behandelnden ab.

## Formulierung 1

| Tablette | 10 mg Tab. | 25 mg Tab. | 100 mg Tab. |
|---|---|---|---|
| a) 1-N-(2,3-Dihydroxypropyloxy-carbonyl)sisomicin | $10{,}50^{+}$ mg | $26{,}25^{+}$ mg | $105{,}00^{+}$ mg |
| Lactose | 197,50 mg | 171,25 mg | 126,00 mg |
| Maisstärke | 25,00 mg | 25,00 mg | 35,00 mg |
| Polyvinylpyrrolidon | 7,50 mg | 7,50 mg | 7,50 mg |
| Magnesiumstearat | 2,50 mg | 2,50 mg | 3,50 mg |

$^{+}$ 5%iger Überschuß

| Tablette | 10 mg Tab. | 25 mg Tab. | 100 mg Tab. |
|---|---|---|---|
| b) 1-N-(Hydroxycarbamoyl)-sisomicin | 10,50+ mg | 26,25+ mg | 105,00+ mg |
| Lactose | 197,50 mg | 171,25 mg | 126,00 mg |
| Maisstärke | 25,00 mg | 25,00 mg | 35,00 mg |
| Polyvinylpyrrolidon | 7,50 mg | 7,50 mg | 7,50 mg |
| Magnesiumstearat | 2,50 mg | 2,50 mg | 3,50 mg |

+ 5%iger Überschuß

Zur Herstellung der Tabletten wird eine Aufschlämmung von 1-N-(2,3-Dihydroxypropyloxycarbonyl)-sisomicin bzw. 1-N-(Hydroxycarbamoyl)-sisomicin, Lactose und Polyvinylpyrrolidon, hergestellt und diese sprühgetrocknet. Man gibt die Maisstärke und Magnesiumstearat zu und verpreßt zu Tabletten.

## Formulierung 2

Salbe

| | |
|---|---|
| 1-N-(2,3-Dihydroxypropyloxycarbonyl)-sisomicin | 1,0 g |
| Methylparaben U.S.P. | 0,5 g |
| Propylparaben U.S.P. | 0,1 g |
| Petrolatum | auf 1000 g |

Herstellung

(1) Man schmilzt das Petrolatum;

(2) Man mischt 1-N-(2,3-Dihydroxypropyloxycarbonyl)-sisomicin, Methylparaben und Propylparaben mit ca. 10% des geschmolzenen Petrolatum;

(3) Gibt das Gemisch in eine Kolloidmühle;

(4) Gibt das restliche Petrolatum unter Rühren zu und kühlt bis es halbfest wird. Das Produkt wird in geeignete Behälter abgefüllt.

## Formulierung 3

| Injektionslösung | Per 2,0 ml Phiole | per 50 Liter |
|---|---|---|
| 1-N-(2,3-Dihydroxypropyloxycarbonyl)sisomicin | 84,0 mg+ | 2100,0 gm |
| Mathylparaben, U.S.P. | 3,6 mg | 90,0 gm |
| Propylparaben, U.S.P. | 0,4 mg | 10,0 gm |
| Natriumbisulfit, U.S.P. | 6,4 mg | 160,0 gm |
| Dinatriumäthylendiamintetraacetat-dihydrat | 0,2 mg | 5,0 gm |
| Wasser, U.S.P. q.s. | 2,0 mg | 50,0 Liter |

+5%iger Überschuß

Die mit a versehenen Beispiel beziehen sich auf die Herstellung von Ausgangsverbindungen.

Beispiel 1a

*4-Aethoxycarbonyloxy-3-oxo-2,5-diphenyl-2,3-dihydrothiophen-1,1-dioxid*

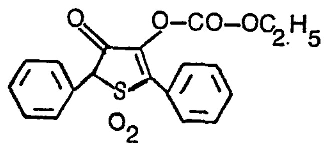

2 g 4,6-Diphenyl-thieno[3,4-d][1,3]-dioxol-2-on-5,5-dioxid (Angew. Chem. *88* 480 (1976)) werden in 40 ml Aethanol bis zum Verschwinden der gelben Farbe erhitat. Nach dem Abkühlen isoliert man 1,2 g 4-Aethoxycarbonyloxy-3-oxo-2,5-diphenyl-2,3-dihydrothiophen-1,1-dioxid, das durch eine Spur 4-Hydroxy-3-oxo-2,5-diphenyl-2,3-dihydrothiophen-1,1-dioxid verunreinigt ist; Schmelzpunkt 132—133°C (Zersetzung).

$C_{19}H_{16}O_6S$ (372,4): Bor.: C 61,28 H 4,33
Gef.: 61,4 4,6

IR (KBr): 1723, 1772 $cm^{-1}$;

NMR ($CDCl_2$): $\delta$ 1,34 t ($CH_3$), 4,3 q (O—$CH_2$), 5,17 s (CH), 7,1—7,65 m (8 aromatische Protonen), 7,7—8,15 m (2 aromatische Protonen) [ppm];

MS (70 eV): m/e 328 (372—$CO_2$), 300 (372—$CO_2C_2H_4$), 264.

Analog Beispiel la werden die entsprechenden Zwischenprodukte der Tabelle I hergestellt. Hierbei sollte die Umsetzung bei Alkoholen mit einem Siedepunkt > 100°C zweckmäßig in äquivalenten Mengen in einem inerten Lösungsmittel wie Toluol oder Acetonitril durchgeführt werden. Die Reaktionsprodukte enthalten häufig einen mehr oder weniger großen Anteil an 4-Hydroxy-3-oxo-2,5-diphenyl-2,3,-dihydrothiophen-1,1-dioxid, der bei der Folgereaktion nicht stört und daher nicht entfernt zu werden braucht.

TABELLE I

Zwischenprodukte der Formel

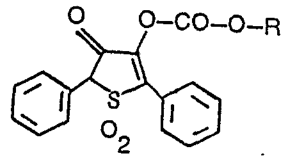

| Beispiel Nr. | R | IR (KBr) ($cm^{-1}$) |
|---|---|---|
| 2a | $CH_3$ | 1730, 1780 |
| 3a | $C_3H_7$ | 1730, 1775 |
| 4a | $C_4H_9$ | 1730, 1775 |
| 5a | $CH(CH_3)_2$ | 1725, 1770, 1785 |
| 6a | $CH_2-CH(CH_3)_2$ | 1728, 1780 |
| 7a | $C(CH_3)_3$ | |
| 8a | 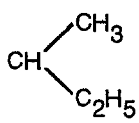 | 1729, 1778 |
| 9a | $C_5H_{11}$ | 1728, 1775 |
| 10a | $CH_2-CH_2-CH(CH_3)_2$ | 1725, 1777 |
| 11a | $CH_2-C(CH_3)_3$ | 1729, 1780 |
| 12a | $(CH_2)_7-CH_3$ | 1728, 1780 |
| 13a | $CH_2CH_2Cl$ | 1729, 1780 |
| 14a | $(CH_2)_4Cl$ | 1720, 1777 |
| 15a | $CH_2CH_2OCH_3$ | 1730, 1782 |
| 16a | $CH_2CH_2CN$ | 1725, 1780, 2260 |
| 17a | 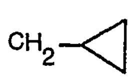 | 1728, 1778 |
| 18a | 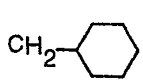 | 1730, 1778 |
| 19a | 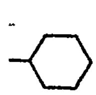 | 1730, 1770 |
| 20a | 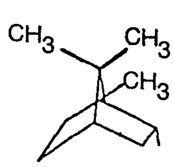 | 1725, 1770, 1730, 1775 |

TABELLE I (Fortsetzung)

| Beispiel Nr. | R | IR (KBr) ($cm^{-1}$) |
|---|---|---|
| 21a | $CH_2-CH=CH_2$ | 1728, 1778 |
| 22a | $CH_2-C\equiv CH$ | 1731, 1787 |
| 23a | $CH_2-CH_2-O-C_4H_5$ | 1730, 1775 |
| 24a | $CH_2-C_6H_5$ | |
| 25a | 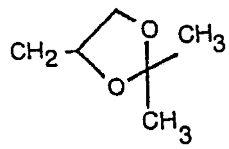 | 1725, 1778 |
| 26a | 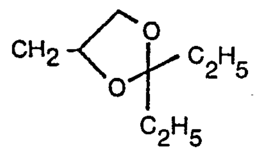 | 1729, 1780 |
| 27a | 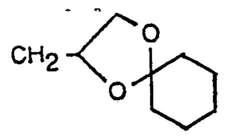 | 1730, 1780 |
| 28a | 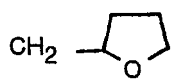 | 1728, 1778 |
| 29a | 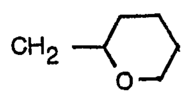 | 1730, 1782 |
| 30a | 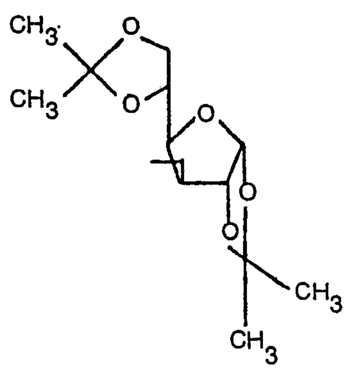 | 1725, 1780 |
| 31a | 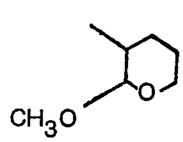 | 1730, 1780 |

TABELLE I (Fortsetzung)

| Beispiel Nr. | R | IR (KBr) ($cm^{-1}$) |
|---|---|---|
| 32a | 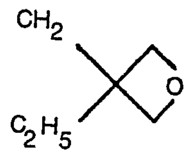 | 1730, 1782 |
| 33a | 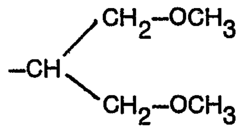 | 1730, 1783 |
| 34a | 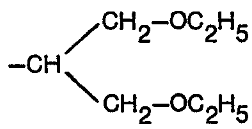 | 1730, 1789 |
| 35a | $CH_2-CO_2-C_4H_6$ | 1729, 1753, 1790 |
| 36a | 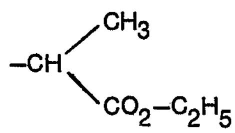 | 1729, 1733, 1786 |
| 37a | 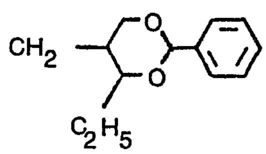 | 1730, 1781 |
| 38a | $CH_2CH_2-S-CH_3$ | 1730, 1782 |
| 39a | 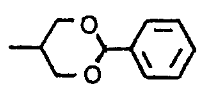 | 1732, 1780 |
| 40a | 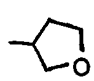 | 1728, 1778 |

Beispiel 41a

*(4-Nitrophenyl)-[2-(2-nitrophenylsulfenyl-methyl-amino)-äthyl]-carbonat*

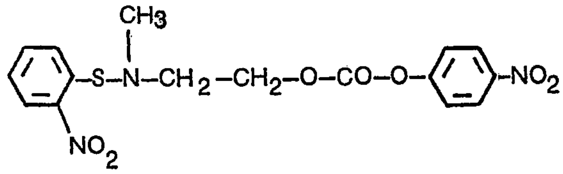

Man legt eine Lösung von 1,4 g 2-Methylamino-äthanol in 30 ml Dioxan vor und tropft gleichzeitig unter Aufrechterhaltung von pH 8 eine Lösung von 3,8 g o-Nitrophenylsulfensäurechlorid in 10 ml Dioxan und 8,5 ml 2n-Natronlauge zu. Nach mehrstündigem Rühren bei Raumtemperatur wird im Vakuum eingeengt, der Rückstand in Essigester aufgenommen und zweimal mit Wasser gewaschen, mit $Na_2SO_4$ getrocknet und im Vakuum eingeengt. Das zurückbleibende Oel wird mit Toluol/Essigester (2:1) über 100 g Kieselgel chromatographiert und die Hauptkomponente ($R_f$ : 0,29) abgetrennt. Ausbeute: 2,9 g N-(2-Hydroxyäthyl)-N-methyl-o-nitrosulfensäureamid; Schmelzpunkt: 53—56°C.

456 mg dieser Verbindung und 600 mg Chlorameisensäure-p-nitrophenylester werden in 5 ml Acetonitril gelöst und unter Eiskühlung mit 300 mg Triäthylamin in 5 ml Acetonitril versetzt. Nach 1 Stunde bei Raumtemperatur wird im Vakuum eingeengt, in 30 ml Methylenchlorid aufgenommen, zweimal mit Wasser gewaschen, mit $Na_2SO_4$ getrocknet, in Vakuum eingeengt und das erhaltene orange Ocl mit Toluol/Essigester (2:1) über 100 g Kieselgel chromatographiert und die Hauptfraktion abgetrennt. Ausbeute: 250 mg oranges Oel, das langsam durchkristallisiert. IR (KBr): 1770 $cm^{-1}$. $R_f$-Wert (Toluol/Essigester 2:1): 0,84.

Beispiel 42a

*(4-Nitrophenyl)-[2-(2-nitrophenylsulfenylamino)-äthyl]-carbonat*

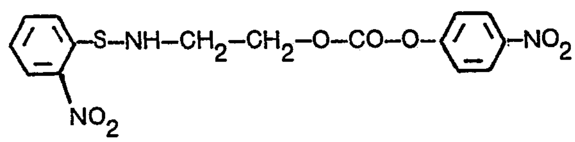

Man verfahrt Beispiel 41A IR: 1770 $cm^{-1}$; $R_f$-Wert(Toluol/Essigester 2:1): 0,77.

Beispiel 43a

*(4-Nitrophenyl)-[2-(2-nitrophenylsulfenylamino)-cyclohexyl]-carbonat*

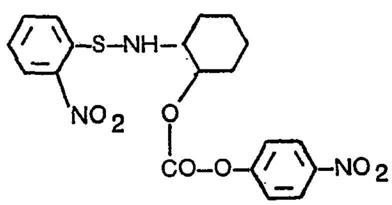

Man verfahrt analog Beispiel 41a; IR: 1770 $cm^{-1}$, $R_f$-Wert (Toluol/Essigester 2:1) 0.92 (cis-trans-Gemisch nach NMR).

Die in folgender Tabelle Ia aufgeführten Beispiele werden entsprechend Beispiel 41A erhalten:

TABELLE Ia

Zwischenprodukte der Formel

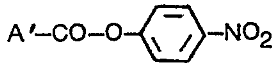

| Beispiel Nr. | A' | IR ($cm^{-1}$) |
|---|---|---|
| 44a | 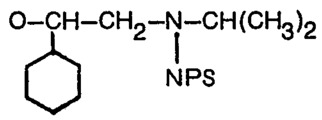 | 1772 |
| 45a | $O-CH_2$ $CH_2-N-C_3H_7$<br>NPS | 1768 |
| 46a | $O-CH_2$ $CH_2-N-C_4H_9$<br>NPS | 1770 |
| 47a | $O-CH_2$ $CH_2-N-CH_2CH(CH_3)_2$<br>NPS | 1769 |
| 48a | $O-CH$ $CH_2-N-CH_3$<br>$CH_3$ NPS | 1766 |
| 49a | $O-CH_2CH_2-CH-NH-NPS$<br>$CH_3$ | 1766 |
| 50a | $CH_3$<br>$O-CH-CH_2-C-NH-NPS$<br>$CH_3$ $CH_3$ | 1763 |
| 51a | $O-CH-CH_2-N-CH(CH_3)_2$<br>$CH_3$ NPS | 1763 |
| 52a | $O-CH_2CH_2-N-(CH_2)_5-CH_3$<br>NPS | 1767 |
| 53a | $O-CH_2CH_2-N-C(CH_3)_3$<br>NPS | 1762 |
| 54a | $O-(CH_2)_3-NH-NPS$ | 1763 |
| 55a | $O-(CH_2)_4-NH-NPS$ | 1769 |
| 56a | $O-(CH_2)_5-NH-NPS$ | 1768 |
| 57a | $O-(CH_2)_6-NH-NPS$ | 1770 |

TABELLE Ia (Fortsetzung)

| Beispiel Nr. | A' | IR ($cm^{-1}$) |
|---|---|---|
| 58a | 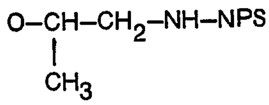 | 1770 |
| 59a | $O-CH_2CH_2OCH_2CH_2-NH-NPS$ | 1768 |
| 60a | $O-CH_2CH_2O(CH_2)_3-NH-NPS$ | 1770 |
| 61a | $O-CH_2CH_2SCH_2CH_2-NH-NPS$ | 1768 |
| 62a | 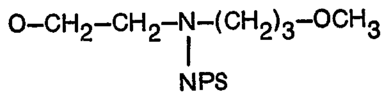 | 1770 |
| 63a | 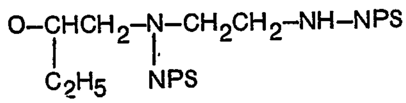 | 1765 |
| 64a | 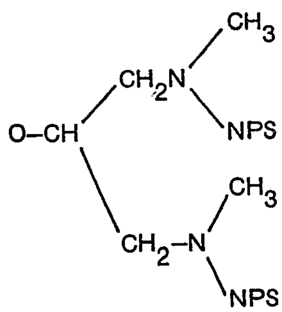 | 1760 |
| 65a | 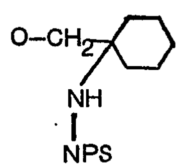 | 1780 |
| 66a | 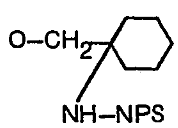 | 1770 |
| 67a | 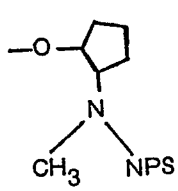 | 1767 |
| 68a | 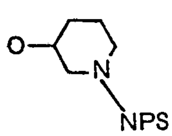 | 1765 |

TABELLE Ia (Fortsetzung)

| Beispiel Nr. | A′ | IR ($cm^{-1}$) |
|---|---|---|
| 69a | 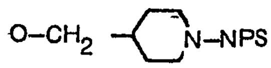 | 1760 |
| 70a | 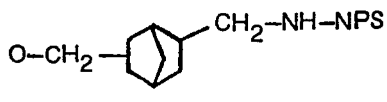 | 1768 |
| 71a | 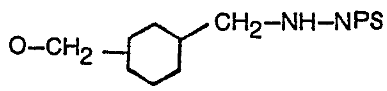 | 1765 |
| 72a | 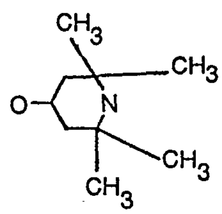 | 1765 |
| 73a | 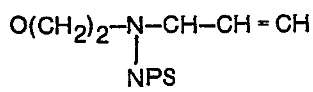 | 1763 |
| 74a | 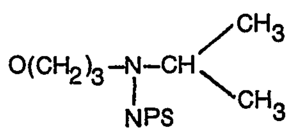 | 1763 |
| 75a | 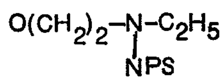 | 1761 |
| 76a | 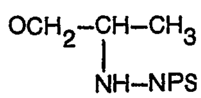 | 1762 |
| 77a | 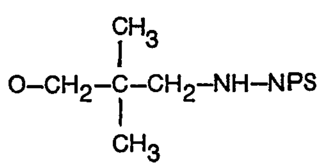 | 1762 |
| 78a | 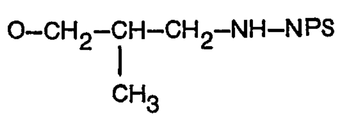 | 1770 |
| 79a | 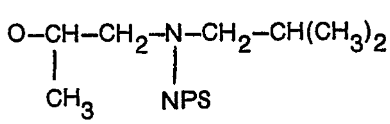 | 1761 |
| 80a | 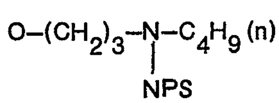 | 1763 |

TABLE Ia (Fortsetzung)

| Beispiel Nr. | A' | IR ($cm^{-1}$) |
|---|---|---|
| 81a | 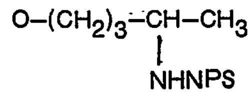 | 1768 |
| 82a | 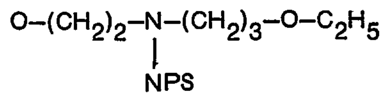 | 1769 |
| 83a | 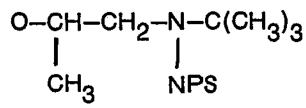 | 1762 |
| 84a | 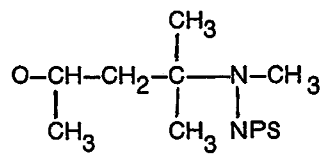 | 1764 |
| 85a | 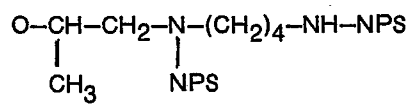 | 1761 |
| 86a | 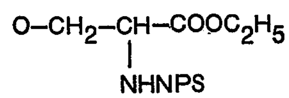 | 1762 |
| 87a | 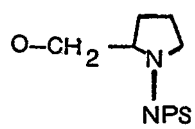 | 1763 |

Beispiel 88a

*4-Nitrophenyl-(2-triphenylmethyloxyäthyl)-carbonat*

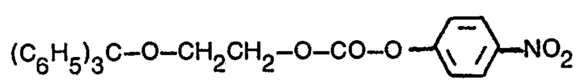

921 mg O-Monotriphenylmethylglykol (J. Chem. Soc. *1960*, 2587) werden in 5 ml Pyridin mit 630 mg Chlorameisensäure-p-nitrophenylester versetzt, 30 Minuten bei Raumtemperatur gerührt und das Reaktionsgemisch eingeengt. Nach Chromatographie an 50 g Kieselgel mit Toluol als Laufmittel erhält man 600 mg 4-Nitrophenyl-(2-triphenylmethyloxyäthyl)-carbonat vom Schmelzpunkt 136—141°C; IR: 1775 $cm^{-1}$

Beispiel 89a

*4-Nitrophenyl-(2-triphenylmethyloxyäthyloxyäthyl)-carbonat*

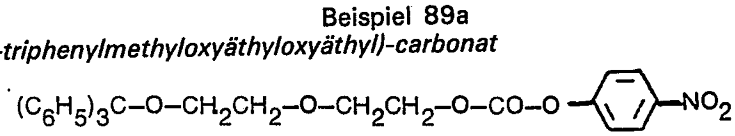

Man verfährt analog Beispiel 88a; Ausbeute: 0,9 g Öl, IR: 1770 $cm^{-1}$.

Beispiel 90a

*4-Nitrophenyl-(2-dimethylamino-äthyl)-carbonat-hydrochlorid*

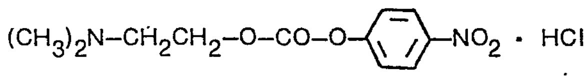

270 mg 2-Dimethylaminoäthanol werden in 5 ml Acetonitril mit 630 mg Chlorameisensäure-p-nitrophenylester 30 Minuten bei Raumtemperatur gerührt, im Vakuum eingeengt, mit 2 ml Essigester verrührt; Ausbeute: 0,4 g; IR: 1770 $cm^{-1}$.

Beispiel 91a

*4-Nitrophenyl-(3-dimethylaminopropyl)-carbonat-hydrochlorid*

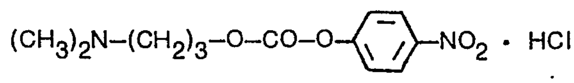

Man verfährt analog Beispiel 90a; IR: 1768 $cm^{-1}$.

Beispiel 92a

*4-Nitrophenyl-(2-diäthylaminoäthyl)-carbonat-hydrochlorid*

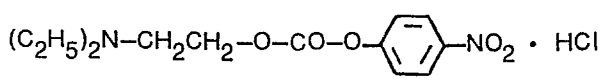

Man verfährt analog Beispiel 90a, IR: 1760 $cm^{-1}$.

Beispiel 1

*1-N-Isoproxycarbonyl-sisomicin*

110 mg 2', 3, 3'', 6'-Tetra-N-(o-nitrophenylsulfenyl)-sisomicin ($10^{-4}$ Mol) werden in 0,5 ml Pyridin mit 50 mg 4-Isopropoxycarbonyloxy-3-oxo-2,5-diphenyl-2,3-dihydrothiophen-1,1-dioxid ($1,3 \times 10^{-4}$ Mol) versetzt und einen Tag bei Raumtemperatur stehen gelassen. Danach wird im Vakuum eingeengt, der Rückstand in 0,3 ml Methylenchlorid/Methanol (95:5) gelöst und mit Methylenchlorid/Methanol (95:5) über eine Kieselgelsäule filtriert. Dadurch wird das Acylierungsprodukt von einem am Start verbleibenen Nebenprodukt abgetrennt. Man dampft das Eluat im Vakuum ein, nimmt den Rückstand in 3 ml Methylenchlorid auf, versetzt mit 0,5 ml einer Lösung von 4% Schwefelwasserstoff in Methanol und säuert mit einigen Tropfen einer methanolischen Chlorwasserstofflösung an. Nach 5 Minuten wird zweimal mit je 2 ml Wasser extrahiert, die Extrakte werden mit Methylenchlorid ausgeschüttelt, mit Aktivkohle behandelt und die fast farblose Lösung mit ca. 1 ml eines basischen Ionenaustauschers (Lewatit MP 500) kurz geschüttelt. Man dampft im Hochvakuum ein, nimmt den Rückstand in wenig Methanol auf und behandelt nochmals kurz mit Aktivkohle. Nach dem Einengen Erhält man 19 mg 1-N-Isopropoxycarbonyl-sisomicin; $R_f$-Wert[+)]: 0,61.

Analog Beispiel 1 werden die in Tabelle II aufgeführten Verbindungen erhalten.

+) Falls nicht anders angegeben, werden alle in den Beispielen angegebenen $R_f$-Werte auf 20 × 20-DC-Fertigplatten Kieselgel 60 F—254 (MERCK, Darmstadt) im Laufmittelsystem Dichlormethan/Methanol/20%—Ammoniak (2:4:1) bei Raumtemperatur gegen Sisomicin als Vergleichssubstanz ($R_f = 0,24$) gemessen.

TABELLE II

Verbindungen der Formel

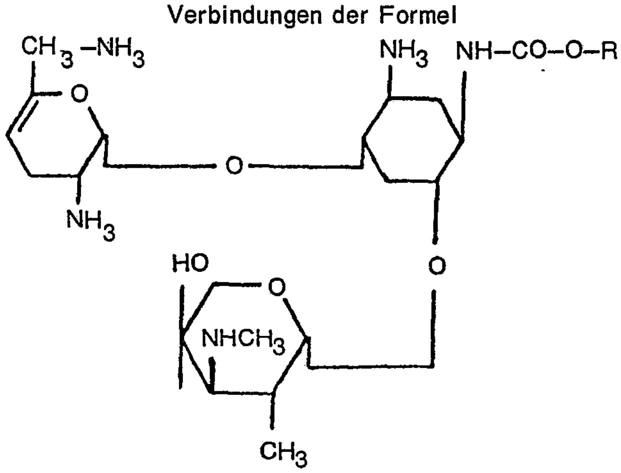

| Beispiel Nr. | R | $R_f$-Wert +) |
|---|---|---|
| 2 | $CH_3$ | 0,54 |
| 3 | $C_2H_5$ | 0,65 |
| 4 | $C_3H_7$ | 0,63 |
| 5 | $C_4H_6$ | 0,71 |
| 6 | $CH_2CH(CH_3)_2$ | 0,65 |
| 7 | $C(CH_3)_3$ | 0,79 |
| 8 | $CH(C_2H_5)(CH_3)$ | 0,70 |
| 9 | $C_5H_{11}$ | 0,67 |
| 10 | $CH_2CH_2CH(CH_3)_2$ | 0,69 |
| 11 | $CH_2C(CH_3)_3$ | 0,74 |
| 12 | $(CH_2)_7CH_3$ | 0,72 |
| 13 | $CH_2CH_2Cl$ | 0,57 |
| 14 | $(CH_2)_4Cl$ | 0,63 |
| 15 | $CH_2CH_2-OCH_3$ | 0,65 |
| 16 | $CH_2$- 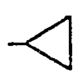 | 0,62 |
| 17 | $CH_2$- 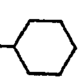 | 0,67 |
| 18 | 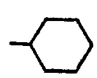 | 0,75 |

+) vgl. Beispiel 1.

TABLE II (Fortsetzung)

| Beispiel Nr. | R | $R_f$-Wert |
|---|---|---|
| 19 | 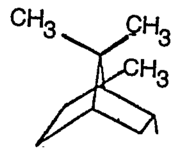 | 0,75 |
| 20 | $CH_2-CH=CH_2$ | 0,72 |
| 21 | $CH_3-C\equiv CH$ | 0,55 |
| 22 | $CH_2CH_2-O-C_6H_5$ | 0,67 |
| 23 | $CH_2-C_6H_5$ | 0,65 |
| 24 | 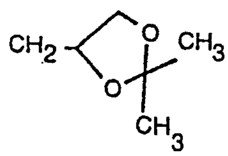 | 0,55 |
| 25 | 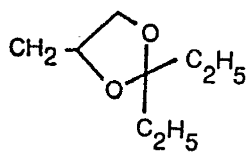 | 0,74 |
| 26 | 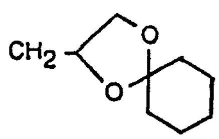 | 0,71 |
| 27 | 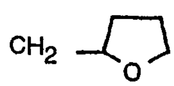 | 0,60 |
| 28 | 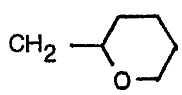 | 0,60 |
| 29 | 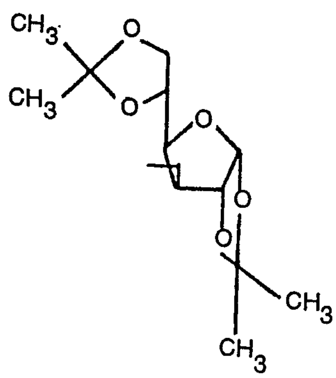 | 0,56 |

TABLE II (Fortsetzung)

| Beispiel Nr. | R | $R_f$-Wert |
|---|---|---|
| 30 | 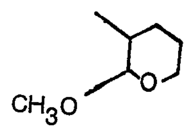 | 0,68 |
| 31 | 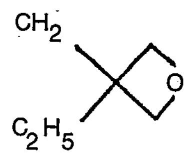 | 0,67 |
| 32 | $CH(CH_2OCH_3)_2$ written as CH with branches $CH_2OCH_3$ / $CH_2OCH_3$ | 0,66 |
| 33 | CH with branches $CH_2OC_2H_5$ / $CH_2OC_2H_5$ | 0,75 |
| 34 | $CH_2-CO_2-C_4H_6$ | |
| 35 | CH with branches $CH_3$ / $CO_2C_2H_5$ | 0,50 |
| 36 | $CH_2CH_2-NH_2$ | 0,28 |
| 37 | $CH_2CH_2-NH-CH_3$ | 0,30 |
| 38 | 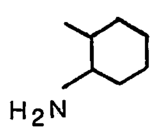 | 0,28 |
| 39 | $CHCH_2-NH-CH(CH_3)_2$ 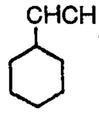 | 0,62 |
| 40 | $CH_2CH_2-NH-C_3H_7$-n | 0,46 |
| 41 | $CH_2CH_2-NH-C_4H_9$-n | 0,50 |
| 42 | $CH_2CH_2-NH-CH_2CH(CH_3)_2$ | 0,54 |
| 43 | $CHCH_2-NH-CH_3$ with $CH_3$ on CH | 0,28 |
| 44 | $CH_2CH_2CH-NH_2$ with $CH_3$ on CH | 0,27 |

TABELLE II (Fortsetzung)

| Beispiel Nr. | R | $R_f$-Werte |
|---|---|---|
| 45 | 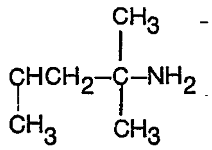 | 0,36 |
| 46 | 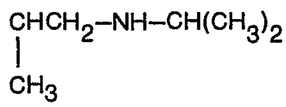 | 0,52 |
| 47 | $CH_2CH_2{-}NH{-}(CH_2)_5CH_3$ | 0,56 |
| 48 | $CH_2CH_2{-}NH{-}C(CH_3)_3$ | 0,56 |
| 49 | $(CH_2)_3{-}NH_2$ | 0,20 |
| 50 | $(CH_2)_4{-}NH_2$ | 0,18 |
| 51 | $(CH_2)_5{-}NH_2$ | 0,25 |
| 52 | $(CH_2)_6{-}NH_2$ | 0,23 |
| 53 | 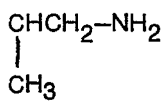 | 0,49 |
| 54 | $CH_2CH_2OCH_2CH_2{-}NH_2$ | 0,30 |
| 55 | $CH_2CH_2O(CH_2)_3{-}NH_2$ | 0,15 |
| 56 | $CH_2CH_2SCH_2CH_2{-}NH_2$ | 0,38 |
| 57 | $CH_2CH_2{-}NH{-}(CH_2)_3{-}OCH_3$ | 0,47 |
| 58 | 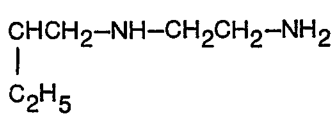 | 0,35 |
| 59 | 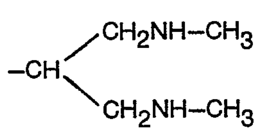 | 0,16 |
| 60 | 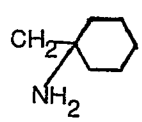 | 0,49 |
| 61 | 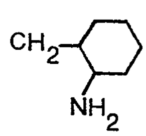 | 0,43 |

TABELLE II (Fortsetzung)

| Beispiel Nr. | R | $R_f$-Werte |
|---|---|---|
| 62 | 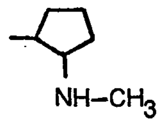 | 0,43 |
| 63 | 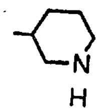 | 0,28 |
| 64 | 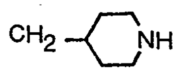 | 0,16 |
| 65 | 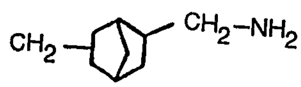 | 0,49 |
| 66 | 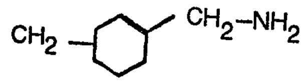 | 0,35 |
| 67 | 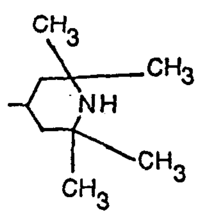 | 0,57 |
| 68 | $(CH_2)_2-NH-CH_2-CH=CH_2$ | 0,56 |
| 69 | $(CH_2)_3-NH-CH(CH_3)_2$ | 0,51 |
| 70 | $(CH_2)NH-C_2H_5$ | 0,43 |
| 71 | $-CH_2-\underset{\displaystyle NH_2}{\underset{\vert}{CH}}-CH_3$ | 0,48 |
| 72 | $-CH_2-C(CH_3)_2-CH_2-NH_2$ | 0,50 |
| 73 | $-CH_2-CHCH_3-CH_2-NH_2$ | 0,29 |
| 74 | $-CHCH_3-CH_2-NH-CH_2-CH(CH_3)_2$ | 0,61 |
| 75 | $-(CH_2)_3-NH-C_4H_9$ (n) | 0,54 |

TABELLE II (Fortsetzung)

| Beispiel Nr. | R | $R_f$-Werte |
|---|---|---|
| 76 | 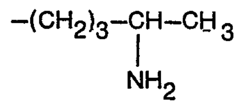 | 0,32 |
| 77 | $-(CH_2)_2-NH-(CH_2)_3-O-C_2H_5$ | 0,53 |
| 78 | $-CHCH_3-CH_2-NH-C(CH_3)_3$ | 0,64 |
| 79 | $-CHCH_3-CH_2-C(CH_3)_2-NH-CH_3$ | 0,34 |
| 80 | $-CHCH_3-CH_2-NH-(CH_2)_3-NH_2$ | 0,10 |
| 81 | 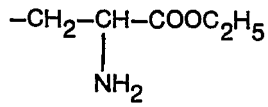 | 0,47 |
| 82 | 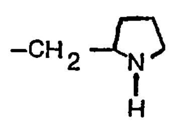 | 0,30 |
| 83 | $CH_2CH_2-N(CH_3)_2$ | 0,40 |
| 84 | $CH_2CH_2-N(C_2H_5)_2$ | 0,46 |
| 85 | $CH_2CH_2CH_2-N(CH_3)_2$ | 0,37 |
| 86 | $CH_2CH_2-SCH_3$ | 0,56 |
| 87 | 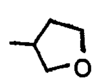 | 0,52 |

Beispiel 88

*1-N-Aethoxycarbonyl-sisomicin*

220 mg 2',3,3",6'-Tetra-N-(o-nitrophenylsulfenyl)-sisomicin in 4 ml Dichlormethan und 2 ml Methanol werden mit 40 mg Pyrokohlensäurediäthylester versetzt, das Reaktionsgemisch nach 1 Stunde im Vakuum eingedampft, die Schutzgruppe wie in Beispiel 1 beschrieben abgespalten und der Ansatz aufgearbeitet. Ausbeute: 78mg; $R_f$-Wert: 0,65.

Beispiel 89

*1-N-Aethoxycarbonyl-sisomicin*

Man arbeitet analog Beispiel 1 mit Chlorameisensäureäthylester als Acylierungsmittel. Ausbeute: 20 mg; $R_f$-Wert: 0.65.

Beispiel 90

*1-N-tert-Butoxycarbonyl-sisomicin*

Die Darstellung erfolgt analog Beispiel 88 mit Pyrokohlensäuredi-(tert-butylester) als Acylierungsmittel. Ausbeute: 75 mg; $R_f$-Wert: 0,79.

Beispiel 91

*1-N-tert-Butoxycarbonyl-sisomicin*

Man arbeitet analog Beispiel 1 mit tert-Butoxycarbonylazid als Acylierungsmittel. Ausbeute: 23 mg; $R_f$-Wert: 0,79.

Beispiel 92

*1-N-(2,3-Dihydroxypropyloxycarbonyl)-sisomicin*

Man setzt 110 g 2',3,3",6'-Tetra-N-(o-nitrophenylsulfenyl)-sisomicin mit 58 mg 4-(2,2-Diäthyl-1,3-dioxolan-4-yl-methoxycarbonyloxy)-3-oxo-2,5-diphenyl-2,3-dihydrothiophen-1,1-dioxid (aus Beispiel 26a) analog Beispiel 1 um. Zur Abspaltung der Schutzgruppen sauert man auf pH 1 an und laßt 10—15 Minuten stehen. Danach wird wie in Beispiel 1 aufgearbeitet. Ausbeute: 10 mg; $R_f$-Wert: 0,36.

Beispiel 93

a) *1-N-(p-Nitrophenoxycarbonyl)-2',3,3",6'-tetra-N-(o-nitro-phenylsulfenyl)-sisomicin*

617 mg Chlorameisensäure-p-nitrophenylester in 25 ml absolutem Dichlormethan werden unter Eiskühlung mit 1,23 ml absolutem Pyridin und nach 2 Minuten mit 1,8 g 2',3,3",6'-Tetra-N-(o-nitrophenylsulfenyl)-sisomicin versetzt und unter Feuchtigkeitsausschluß kraftig gerührt. Nach 5 Minuten wird die rote Lösung auf 200 mg Aether gegossen, der Niederschlag abgesaugt, in 40 ml Dichlormethan gelost, zweimal mit je 150 ml Wasser ausgeschuttelt, die organische Phase nach Trocknen über Natriumsulfat auf 15 ml eingeengt und über eine Kieselgel-Säule (3 x 8 cm) filtiert. Nach Eluieren der gelben Zone mit Dichlormethan wird die dunkelrote Fraktion mit Dichlormethan/Methanol (98/2) eluiert und im Vakuum eingedampft. Ausbeute: 1,2 g.

IR (KBr): 1740 (m), 1510 (s), 1358 (s);

$R_f$: ($CH_2Cl_2/CH_3OH = 97,5/2,5$):0,32.

Für die Folgeumsetzungen mit Aminoverbindungen ist die säulenchromatographische Reinigung nicht erforderlich.

b) *1-N-(hydroxycarbamoyl)-sisomicin*

135 mg 1-N-(p-Nitrophenoxycarbonyl)-2',3,3",6'-tetra-N-(o-nitrophenylsulfenyl)-sisomicin in 1,5 ml Dioxan werden mit 0,5 ml einer 1-molaren wäßrig-methanolischen Hydroxylamin-Lösung versetzt, nach 3 Stunden im Vakuum eingedampft, der Rückstand in 2 ml Dichlormethan aufgenommen un mit 3 ml einer methanolischen, bei 0°C gesättigten $H_2S$-Lösung sowie mit 0,2 ml einer bei 20°C gesättigten methanolischen Chlorwasserstoff-Lösung versetzt. Nach einer Minute wird mit konzentrierter Ammoniak-Lösung neutralisiert, im Vakuum eingedampft, der Rückstand mit 5 ml Wasser digeriert und der Ansatz filtriert. Das Filtrat wird zweimal mit Aether gewaschen, die wäßrige Phase über 5 ml basischen Ionenaustauscher ($OH^-$ -Form) filtriert und im Vakuum eingedampft. Ausbeute: 34 mg farbloses Pulver; $R_f$: 0,19

Die Verbindungen der Tabelle III wurden in analoger Weise durch Umsetzung von 1-N-(p-Nitrophenoxy-carbonyl)-2',3,3",6'-tetra-N-(o-nitrophenylsulfenyl)-sisomicin mit den entsprechenden Aminen, Hydrazinen oder Hydroxylaminen erhalten. Dabei wurden die Umsetzungen bevorzugt mit den freien Basen in Dichlormethan, Dioxan, Pyridin oder DMF durchgeführt. Salze der eingesetzten Aminoverbindungen wurden vor der Reaktion mit eines geeigneten Ionenaustauscher oder mit Natriummethylat im Methanol behandelt, um die freien Basen zu erhalten.

TABELLE III

Verbindungen der Formel II

| Beispiel Nr. | A | $R_f$-Wert [+] |
|---|---|---|
| 94 | $NH_2$ | 0,27 |
| 95 | $NH-CH_3$ | 0,44 |
| 96 | $N(CH_3)_2$ | 0,59 |
| 97 | 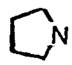 | 0,64 |
| 98 | $NH-CH_2-C\equiv CH$ | 0,46 |
| 99 | $NH-C(CH_3)_3$ | 0,72 |
| 100 | NH– 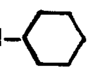 | 0,75 |
| 101 | NH–OH | 0,19 |
| 102 | $NH-(CH_2)_2OH$ | 0,30 |
| 103 | $(NCH_3)-(CH_2)_2OH$ | 0,38 |
| 104 | $NH-CH_2-\underset{OH}{\underset{\mid}{CH}}-CH_2-O-CH_2-CH=CH_2$ | 0,55 |
| 105 | $NH-\overset{CH_3}{\overset{\mid}{CH}}-COOC_2H_5$ | 0,57 |
| 106 | $(NCH_3)-CH_2-CO-NH_2$ | 0,39 |
| 107 | $NH-(CH_2)_3CN$ | 0,52 |
| 108 | $NH-CH_2-CF_3$ | 0,56 |
| 109 | $NH-NH_2$ | 0,19 |
| 110 | $NH-N(CH_3)_2$ | 0,59 |
| 111 | $NH-(CH_2)_2$ - 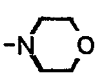 | 0,51 |
| 112 | $NH-O-CH_3$ | 0,47 |
| 113 | $NH-(CH_2)_2-N(CH_3)_2$ | 0,25 |

[+] siehe Beispiel 1.

Beispiel 114

*1-N-(Methylcarbamoyl)-sisomicin*

220 mg 2',3,3",6'-Tetra-N-(o-nitrophenylsulfenyl)-sisomicin werden mit 2 ml einer Lösung von 0,6 ml Methylisocyanat in 10 ml absol. Pyridin versetzt, der Ansatz nach 15 Stunden auf 20 ml Aether gegossen, der Niederschlag abgesaugt, in 3 ml Dichlormethan gelost und mit 6 ml einer methanolischen bei 0°C gesättigten $H_2S$-losung sowie mit 0,4 ml einer bei 20°C gesattigten methanolischen Chlorwasserstofflösung versetzt. Nach 2 Minuten wird mit konzentrierter Ammoniaklösung neutralisiert, der Ansatz im Vakumm eingedampft, mit 20 ml Wasser digeriert und filtriert. Das Filtrat wird zweimal mit Aether gewaschen, über 10 ml basischen Ionenaustauscher ($OH^-$-Form) filtriert und das Eluat im Vakuum eingedampft. Ausbeute: 95 mg; $R_f$-Wert: 0,44.

Auf diese Weise werden mit den entsprechenden Isocyanaten, die in Tabelle IV aufgeführten Verbindungen dargestellt.

TABELLE IV

Verbindungen der Formel II

| Beispiel Nr. | A | $R_f$-Werte + |
|---|---|---|
| 115 | $CH_3-O-CH_2NH$ | 0,46 |
| 116 | $C_2H_5-O-CH_2NH$ | 0,41 |
| 117 | $CH_3-(CH_2)_5-O-CH_2NH$ | 0,42 |
| 118 | $CH_3O-CONH$ | 0,45 |
| 119 | $C_2H_5O-CONH$ | 0,45 |

+) siehe Beispiel 1.

Beispiel 120

*1-N-(Phenoxycarbonyl-carbamoyl)-2',3,3",6'-tetra-N-(o-nitrophenylsulfenyl)-sisomicin*

440 mg 2',3,3",6'-Tetra-N-(o-nitrophenylsulfenyl)-sisomicin in 4 ml absol. Dichlormethan werden tropfenweise mit 0,41 ml einer Lösung von 0.15 ml Phenoxycarbonyl-isocyanat in 0,85 ml Dichlormethan versetzt, der Ansatz nach 2 Minuten auf 50 ml Aether gegossen, der Niederschlag abfiltriert und getrocknet. Ausbeute: 405 mg. IR (KBr): 1740 (m), 1700 (m), 1510 (s); $R_f$ ($CH_2Cl_2/CH_3OH$ = 95/5): 0,95.

Beispiel 121

*1-N-(Allophanyl)-sisomicin*

120 mg 1-N-(Phenoxycarbonyl-carbamoyl)-2',3,3",6'-tetra-N-(o-nitrophenylsulfenyl)-sisomicin in 2 ml Dichlormethan und 1 ml Methanol werden mit wenigen Tropfen konzentrierten Ammoniaks versetzt und die Lösung wie unter Beispiel 114 beschrieben aufgearbeitet. Ausbeute: 40 mg; $R_f$-Wert; 0,36.

Beispiel 122

*1-N-(4-Hydroxyäthylallophanyl)-sisomicin*

Die Darstellung erfolgt mit Äthanolamin, ansonsten wie in Beispiel 121 beschrieben. Ausbeute: 35 mg: $R_f$-Wert: 0,43.

Beispiel 123

*1-N-(N-Methyl-N-morpholinocarbonyl-carbamoyl)-sisomicin*

110 mg 2',3,3",6'-Tetra-N-(o-nitrophenylsulfenyl)-sisomicin und 40 mg N-Methyl-N-morpholinocarbonyl-(2-chlor-4-nitro-phenyl)-urethan werden in 0,5 ml absol. Pyridin 24 Stdn., bei Raumtemperatur belassen, im Vakuum eingedampft und die Abspaltung der Schutzgruppen und die Aufarbeitung wie im Beispiel 114 vorgenommen. Ausbeute: 38 mg; $R_f$-Wert: 0,62.

Beispiel 124

*1-N-(2-Hydroxyäthyloxycarbonyl)sisomicin*

Man setzt analog Beispiel 1, 165 mg 2',3,3",6'-Tetra-N-(o-nitrophenylsulfenyl)-sisomicin mit 84 mg 4-Nitrophenyl-(2-triphenylmethyloxyäthyl)-carbonat um. Zur zusätzlichen Abspaltung des Tritylrestes, die dünnschichtchromatographisch verfolgt werden kann, läßt man die salzsaure Lösung 15 Minuten bei Raumtemperatur stehen, verdünnt mit Wasser und arbeitet danach wie in Beispiel 1 auf. Ausbeute: 25 mg. $R_f$-Wert: 0,43.

Beispiel 125

*1-N-(1,3-Dihydroxyprop-2-yloxycarbonyl)-sisomicin*

Man arbeitet analog Beispiel 92 mit dem Zwischenprodukt aus Beispiel 39a. $R_f$-Wert: 0,32.

Beispiel 126

*1-N-(2,2-Bis-hydroxymethyl-butyloxycarbonyl)-sisomicin*

Man arbeitet analog Beispiel 92 mit dem Zwischenprodukt aus Beispiel 37a. $R_f$-Wert: 0,50.

Beispiel 127

*1-N-(D-Glucopyranosyl-3-carbonyl)-sisomicin*

Man löst 35 mg des Produktes aus Beispiel 29 in wenig verdünnter Salzsäure und läßt zur Abspaltung der Isopropylidengruppen 2 Stunden bei Raumtemperatur stehen, behandelt mit basischem Ionenaustauscher und dampft ein. Ausbeute: 22 mg. $R_f$-Wert: 0,48.

**Patentansprüche**

1. Verbindungen der Formel

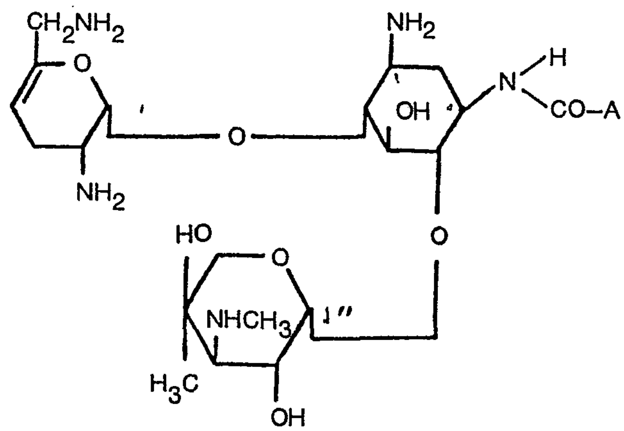

in der

A für OR oder

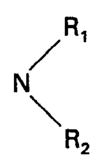

steht, wobei

R einen Alkylrest mit 1 bis 10 C-Atomen, der 1 oder 2 Substituenten aus der Reihe Halogen, Hydroxy, Mercapto, Cyan, Carboxy, Trifluormethyl, Alkoxy sowie Alkythio mit 1 bis 6 Kohlenstoffatomen, das im Alkylteil durch Amino, Monoalkyl- und Dialkylamino mit je 1 bis 4 C-Atomen je Alkylgruppe substituiert sein kann, Allyloxy, Phenoxy,

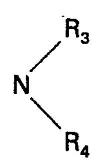 ;

Alkoxycarbonyl mit 1 bis 4 C-Atomen im Alkylrest und Cycloalkyl mit 3 bis Kohlenstoffatomen im Cyclus tragen kann;

einen Alkenylrest mit 3 bis 7 C-Atomen;

einen Alkinylrest mit 3 oder 4 C-Atomen;

einen mono- oder bicyclischen Cycloalkylrest mit 3 bis 7 C-Atomen, der 1, 2 oder 3 Substituenten aus der Reihe Alkyl mit 1 bis 4 C-Atomen; Alkoxy mit 1 bis 4 C-Atomen; Hydroxy; Amino; Alkyl- und Dialkylamino mit 1 bis 4 Kohlenstoffatomen je Alkylgruppe aufweisen kann;
einen Phenyl- oder Benzylrest, der durch Nitro, Halogen oder Alkoxy mit 1 bis 4 C-Atomen substituiert sein kann;
einen Piperidinyl-, Tetrahydropyranyl-, Tetrahydrofuryl-, 1,3-Dioxolanyl- oder 1,3-Dioxolano [a, b] tetrahydrofurylrest, der durch 1 oder 2 Reste aus der Reihe Alkoxy mit 1 bis 4 C-Atomen; Hydroxy; Alkyl mit 1 bis 4 C-Atomen und 2,2-Dimethyl-1,3-dioxolanyl-4 substituiert sein kann, oder
einen 1,3-Dioxolanylalkyl-, Tetrahydrofurylalkyl-, Tetrahydropyranylalkyl-, Oxetanylalkyl-, 1,3-Oxathiolanylalkyl-, 1,3-Dithiolanylalkyl-, 1,4-Dioxaspiro[4,5]-decanyl-, Oxiranylalkyl-, Piperidinylalkyl-, Tetrahydropyridinylalkyl- oder Aziridinylalkylrest, bei dem der Alkylrest 1 bis 4 Kohlenstoffatome enthält, und der 1 oder 2 Substituenten aus der Reihe Alkyl mit 1 bis 4 Kohlenstoffatomen; Phenyl und Alkoxy mit 1 bis 4 Kohlenstoffatomen tragen kann, darstellt,
$R_1$ die Bedeutungen Wasserstoff oder R hat,
$R_2$ für R, Wasserstoff oder eine 1-β-Tetra-O-acetyl-D-glucosyl-, 1-β-D-glucosyl-, Tetrahydropyridinyl Morpholino, Piperidino-, Alkoxy- mit 1 bis 4 C-Atomen, Cyclopentyloxy-, Cyclohexyloxy-, gegebenenfalls durch Halogen substituierte Benzyloxy-, Tetrahydropyranyloxy-, Tetrahydrofuranyloxy- oder Hydroxygruppe steht, oder
$R_1$—$R_2$ unter Einschluß des N-Atoms, an das sie gebunden sind, gemeinsam einen Pyrrolidino-, Piperidino-, Morpholino-, Piperazinyl-4-, Hexamethylenamino-, Isoxazolinyl-2- oder Tetrahydroisoxazinyl-2-rest darstellen, der 1 bis 2 Substituenten aus der Reihe Alkyl mit 1 bis 4 C-Atomen, das durch Hydroxy substituiert sein kann, tragen kann;
$R_3$ für Wasserstoff, $C_1$-$C_8$-Alkyl oder Allyl steht, und
$R_4$ Wasserstoff, $C_1$-$C_8$-Alkyl, $C_5$-$C_7$-Cycloalkyl, $C_5$-$C_7$-Cycloalkyl-$C_1$-$C_4$-alkyl, Phenyl, Phenyl-$C_1$-$C_4$-alkyl oder Allyl bedeutet und wobei die Alkyl-, Cycloalkyl- und Phenylreste $R_3$ und $R_4$ durch einen oder zwei Substituenten aus der Reihe Alkoxy mit 1 bis 4 C-Atomen, Amino, Monoalkyl- und Dialkylamino mit je 1 bis 4 C-Atomen je Alkylgruppe oder Hydroxy substituiert sein können,
sowie deren pharmazeutisch verwendbare Salze.

2. Verbindung nach Anspruch 1, worin

$$A\ O{-}CH_2{-}CH_2{-}NH_2$$

bedeutet.

3. Verfahren zur Herstellung von Verbindungen gemäß Anspruch 1, dadurch gekennzeichnet, daß man Verbindungen der Formel

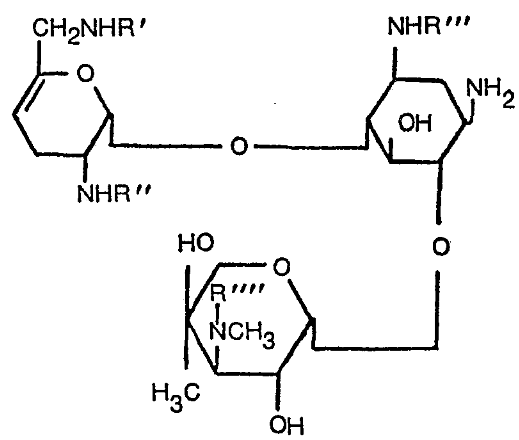

worin
R′, R″, R‴, und R″″ Wasserstoff oder leicht abspaltbare Aminoschutzgruppen bedeuten, oder ihre Salze, mit einem Acylierungsmittel der Formel

$$A'{-}CO{-}E$$

worin
A′ A bedeutet mit der Maßgabe, daß gegebenenfalls in A vorhandene Aminogruppen mit Aminoschutzgruppen blockiert sind und
E für Halogen, $N_3$, OCO—OA′, Phenoxy, 4-Nitrophenoxy, 2,4,5-Trichlorphenoxy oder

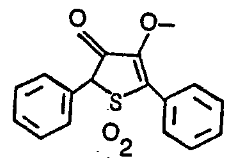

steht,
umsetzt und in üblicher Weise, gegebenenfalls nach Abspaltung von Schutzgruppen, die Produkte isoliert.

4. Verfahren zur Herstellung von Verbindungen gemäß Anspruch 1, worin

A 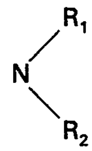

$R_1$ und $R_2$ die in Anspruch 1 genannte Bedeutung haben, dadurch gekennzeichnet, daß man Verbindungen der Formel

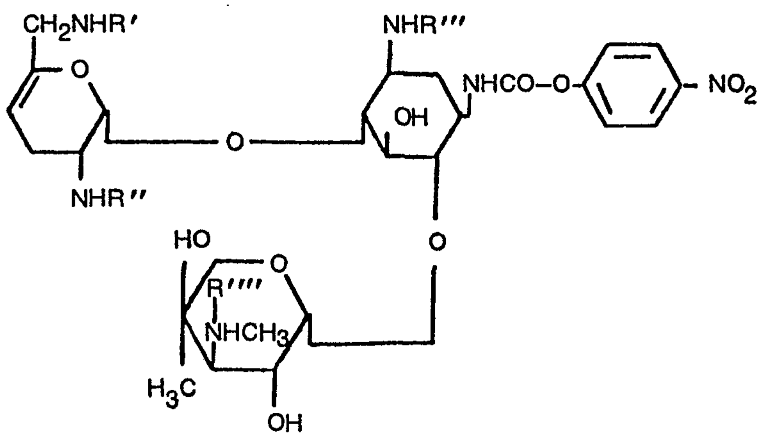

worin
R′, R″, R‴ und R″″ die in Anspruch 3 genannte Bedeutung haben, mit Verbindungen der Formel

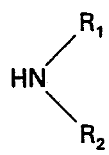 ,

worin
$R_1$ und $R_2$ die obengenannte Bedeutung haben, umsetzt, die Schutzgruppen abspaltet und die gewünschten Produkte isoliert.

5. Verfahren zur Herstellung von Verbindungen gemäß Anspruch 1, worin A NH—$R_2$ bedeutet, dadurch gekennzeichnet, daß man Verbindungen der Formel

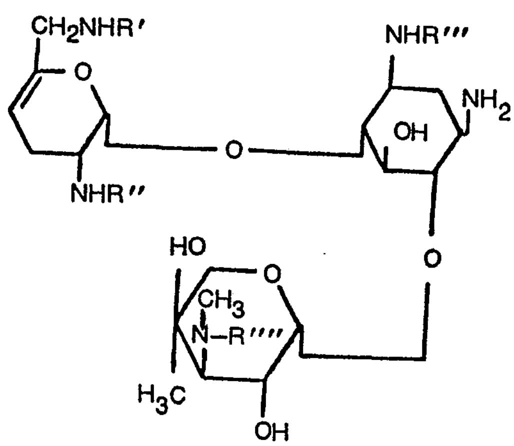

worin
R′, R″, R‴ und R″″ die in Anspruch 3 genannte Bedeutung besitzen, mit Verbindungen der Formel

$$R_2—NCO$$

worin
$R_2$ die in Anspruch 1 genannte Bedeutung besitzt, umsetzt, die Schutzgruppen abspaltet und die gewünschten Produkte isoliert.

6. Arzneimittel enthaltend eine Verbindung gemäß Anspruch 1.

7. Verfahren zur Herstellung eines Arzneimittels, dadurch gekennzeichnet, daß man eine Verbindung gemäß Anspruch 1 mit pharmazeutisch geeigneten Träger- und/oder Zusatzstoffen vermischt.

**Revendications**

1. Composés de formule

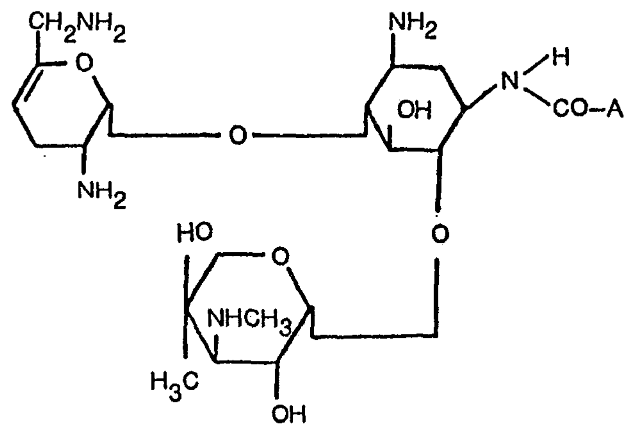

dans laquelle
A représente OR ou

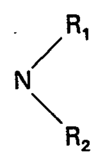

où R représente un reste alkyle contenant de 1 à 10 atomes de carbone, qui peut porter un ou deux substituants pris dans la classe des halogènes, des groupes hydroxy, mercapto, cyano, carboxy, trifluorométhyle, alcoxy ou alkylthio contenant de 1 à 6 atomes de carbone et qui peut être substitué dans la partie alkyle par des groupes amino, monoalkyl- et dialkyl-amino contenant chacun 1 à 4 atomes de carbone par groupe alkyle, allyloxy, phénoxy,

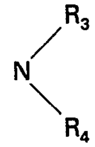 ,

alcoxycarbonyle contenant de 1 à 4 atomes de carbone dans le reste alkyle et cycloalkyle contenant de 3 à 6 atomes de carbone dans le cycle;
un reste alcényle contenant de 3 à 7 atomes de carbone;
un reste alcynyle contenant 3 ou 4 atomes de carbone;
un reste cycloalkyle mono- ou bi-cyclique contenant de 3 à 7 atomes de carbone et qui peut porter 1, 2 ou 3 substituants pris dans la série des groupes alkyle en $C_1—C_4$, alcoxy en $C_1—C_4$, hydroxy-amino, amino, alkyl- et dialkyl-amino contenant 1 à 4 atomes de carbone par groupe alkyle;
un reste phényle ou benzyle qui peut être substitué par des groupes nitro, des halogènes ou des groupes alcoxy en $C_1—C_4$;
un reste pipéridinyle tétrahydropyrannyle, tétrahydrofuryle, 1,3-dioxolannyle ou 1,3-dioxolanno [a,b] tétrahydrofurfuryle qui peut être substitué par un ou deux restes de la série des restes alcoxy en $C_1—C_4$, hydroxy, alkyle en $C_1—C_4$ et 2,2-diméthyl-1,3-dioxolannyle-4, ou bien un reste 1,3-dioxolannylalkyle, tétrahydrofurylalkyle, tétrahydropyrannylalkyle, oxétanylalkyle, 1,3-oxathiolanylalkyle, 1,3-dithiolanylalkyle, 1,4-dioxaspiro [4,5] décanyle, oxiranylalkyle, pipéridinylalkyle, tétrahydropyridinylalkyle ou aziridinylalkyle, dans lequel le reste alkyle contient de 1 à 4 atomes de carbone, et qui peut porter 1 ou 2 substituants de la série des groupes alkyle en $C_1$—$C_4$, phényle et alcoxy en $C_1$—$C_4$,

$R_1$ représente l'hydrogène ou a les significations de R, $R_2$ a les significations de R, représente l'hydrogéne ou un groupe 1-$\beta$-tétra-O-acétyl-D-glucosyle 1-$\beta$-D-glucosyle, tétrahydroyridinyle, morpholino, pipéridino, alcoxy en $C_1$—$C_4$, cyclopentyloxy, cyclohexyloxy, benzyloxy éventuellement substitué par des halogènes, tétrahydropyrannyloxy, tétrahydrofurannyloxy ou hydroxy, ou bein $R_1$—$R_2$ avec l'atome d'azote auquel ils sont reliés, forment en commun un reste pyrrolidino, pipéridino, morpholino, pipérazinyle-4, hexaméthylèneamino, isoxazolinyle-2 ou tétrahydroisoxazinyle-2, qui peut porter 1 ou 2 substituants de la série des groupes alkyle en $C_1$—$C_4$ eux-mêmes éventuellement substitués par des groupes hydroxy;

$R_3$ représente l'hydrogène, un groupe alkyle en $C_1$—$C_8$ ou allyle; et

$R_4$ représente l'hydrogène, un groupe alkyle en $C_1$—$C_8$, cycloalkyle en $C_5$—$C_7$, (cycloalkyle en $C_5$—$C_7$)-alkyle en $C_1$—$C_4$ phényle, phényl-(alkyle en $C_1$—$C_4$) ou allyle, les restes alkyle, cycloalkyle et phényl, $R_3$ et $R_4$ pouvant porter un ou deux substituants de la série des groupes alcoxy en $C_1$—$C_4$, amino, monoalkyl- et dialkyl-amino contenant chacun 1 à 4 atomes de carbone par groupe alkyle, ou hydroxy, et leures sels acceptables pour l'usage pharmaceutique.

2. composé selon la revendication 1, dans lequel A représente O—$CH_2$—$CH_2$—$NH_2$.

3. Procédé de préparation des composés selon la revendication 1, caractérisé en ce que l'on fait réagir des composés de formule

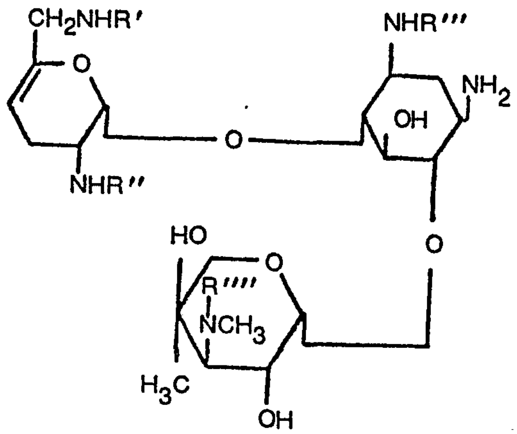

dans laquelle

R', R", R'" et R"" représentent des atomes d'hydrogène ou des groupes protecteurs, faciles à éliminer, des groupes amino, ou leurs sels, avec un agent acylant de formule

A'—CO—E

dans laquelle

A' a la même signification que A, étant spécifié que les groupes amino éventuellement présents dans A sont bloqués par des groupes protecteurs de groupes amino; et

E représente un halogène, $N_3$, OCO—OA', un groupe phénoxy, 4-nitrophénoxy, 2,4,5-trichlorophénoxy ou

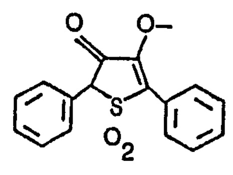

et on isole les produits de la manière habituelle, le cas échéant après élimination des groupes protecteurs.

4. Procédé de préparation des composés selon la revendication 1, dans lesquels

A représente

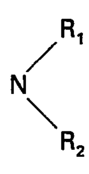

$R_1$ et $R_2$ ayant les significations indiquées dans la revendication 1, caractérisé en ce que l'on fait réagir des composés de formule

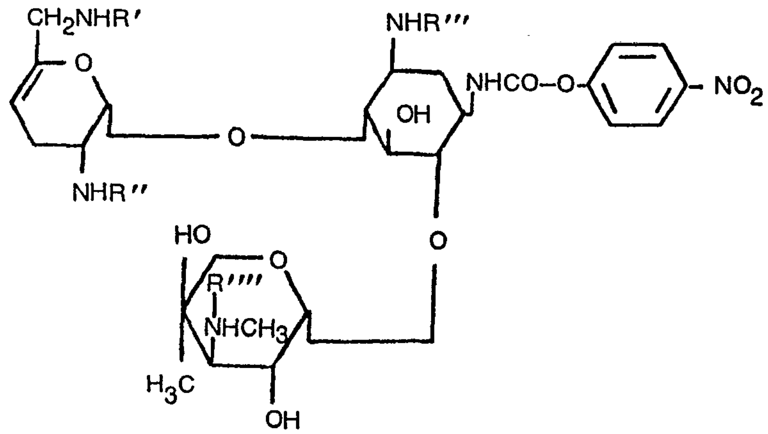

dans laquelle R', R", R"' et R"" ont les significations indiquées dans la revendication 3, avec des composés de formule

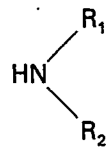

dans laquelle $R_1$ et $R_2$ ont les significations indiquées ci-dessus, on élimine les groupes protecteurs et on isole les produits recherchés.

5. Procédé de préparation des composés selon la revendication 1, dans lesquels A représente NH—$R_2$, caractérisé en cen que l'on fait réagir des composés de formule

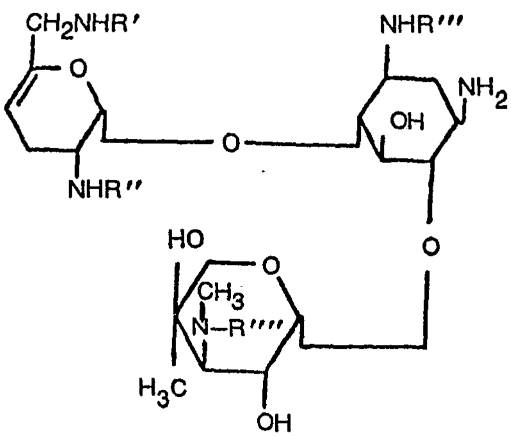

dans laquelle R', R", R"' et R"" ont les significations indiquées dans la revendication 3, avec des composés de formule

$R_2$—NCO

dans laquelle $R_2$ a les significations indiquées dan la revendication 1, on élimine les groupes protecteurs et on isole les produits recherchés.

6. Médicament contenant un composé selon la revendication 1.

7. Procédé de préparation d'un médicament caractérisé en ce qu l'on mélange un composé selon la revendication 1 avec des véhicules et/ou additifs appropriés à l'usage pharmaceutique.

**Claims**

1. Compounds of the formula

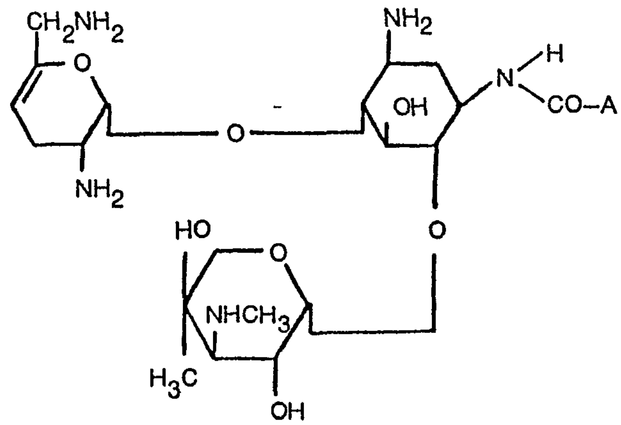

in which
A represents OR or

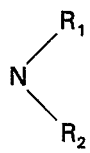
,

wherein R represents an alkyl radical with 1 to 10 C atoms which can carry 1 or 2 substituents from the group comprising halogen, hydroxyl; mercapto; cyano; carboxy; trifluoromethyl; alkoxy and alkylthio with 1 to 6 C atoms, which can be substituted in the alkyl part by amino, monoalkylamino and dialkylamino with 1 to 4 C atomes per alkyle group in each case; allyloxy; phenoxy;

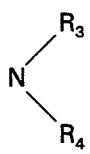
;

alkoxycarbonyl with 1 to 4 C atoms in the alkyl radical and cycloalkyl with 3 to 6 C atoms in the ring; or represents an alkenyl radical with 3 to 7 C atoms; or represents an alkinyl radical with 3 or 4 C atoms; or represents a monocyclic or bicyclic cycloalkyl radical with 3 to 7 C atoms, which can contain 1, 2 or 3 substituents from the group comprising alkyl with 1 to 4 C atoms; alkoxy with 1 to 4 C atoms; hydroxyl; amino; alkylamino and dialkylamino with 1 to 4 carbon atoms per alkyl group; or represents a phenyl or benzyl radical which can be substituted by nitro, halogen or alkoxy with 1 to 4 C atoms; or represents a piperidinyl tetrahydropyranyl, tetrahydrofuryl, 1,3-dioxolanyl or 1,3-dioxolano[a,b]tetrahydrofuryl radical which can be substituted by 1 or 2 radicals from the group comprising alkoxy with 1 to 4 C atoms; hydroxyl; alkyl with 1 to 4 C atoms and 2,2-dimethyl-1,3-dioxolanyl-4, or represents a 1,3-dioxolanylalkyl, tetrahydrofurylalkyl, tetrahydropyranylalkyl, oxetanylalkyl, 1,3-oxathiolanylalkyl, 1,3-dithiolanylalkyl, 1,4-dioxaspiro[4,5]decanyl, oxiranylalkyl, piperidinylalkyl, tetrahydropyridinylalkyl or aziridinylalkyl radical in which the alkyl radical contains 1 to 4 carbon atoms and which can carry 1 or 2 substituents from the group comprising alkyl with 1 to 4 carbon atoms; phenyl and alkoxy with 1 to 4 carbon atoms,

$R_1$ has the meanings hydrogen or R,

$R_2$ represents R, hydrogen, or a 1-β-tetra-O-acetyl-D-glycosyl group, a 1-β-D-glucosyl group, a tetrahydropyridinyl group, a morpholino group, a piperidino group, an alkoxy group with 1 to 4 C atoms, a cyclopentyloxy group, a cyclohexyloxy group, a benzyloxy group optionally substituted by halogen, a tetrahydropyranyloxy group, a tetrahydrofuranyloxy group or a hydroxyl group, or

$R_1$—$R_2$ including the N atom to which they are bonded, together form a pyrrolidino, piperidino, morpholino, piperazin-4-yl, hexamethyleneamino, isoxazolin-2-yl or tetrahydroisoxazin-2-yl radical, which can carry 1 to 2 substituents from the group comprising alkyl with 1 to 4 C atoms, which can be substituted by hydroxyl;

$R_3$ represents hydrogen, $C_1$—$C_8$ alkyl or allyl, and $R_4$ denotes hydrogen, $C_1$—$C_8$ alkyl, $C_5$—$C_7$ cycloalkyl, $C_5$—$C_7$ cycloalkyl-$C_1$—$C_4$-alkyl, phenyl, phenyl-$C_1C_4$-alkyl or allyl and the alkyl, cycloalkyl and phenyl radicals $R_3$ and $R_4$ can be substituted by one or two substituents from the group comprising alkoxy with 1 to 4 C atoms, amino, monoalkylamino and dialkylamino each with 1 to 4 C atoms per alkyl group or hydroxyl, and their pharmaceutically usable salts.

2. Compounds according to claim 1, in which A denotes $O—CH_2—CH_2—NH_2$.

3. Process for the preparation of compounds according to claim 1, characterised in that compounds of the formula

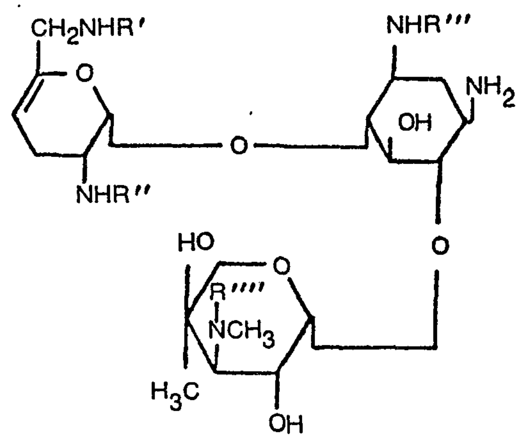

in which
R′, R″, R‴ and R″″ denote hydrogen or amino-protective groups which can be easily split off, or their salts, are reacted with an acylating agent of the formula

A′—CO—E

in which
A′ denotes A with the condition that any amino groups present in A are blocked with amino-protective groups and E represents halogen, $N_3$ OCO—OA′, phenoxy, 4-nitrophenoxy, 2,4,5-trichlorophenoxy or

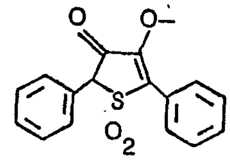

and the products are isolated in the customary manner, if appropriate after splitting off the protective groups.

4. Process for the preparation of compounds according to claim 1, in which

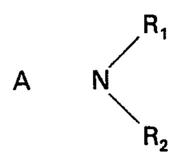

$R_1$ and $R_2$ have the meaning mentioned in claim 1, characterised in that compounds of the formula

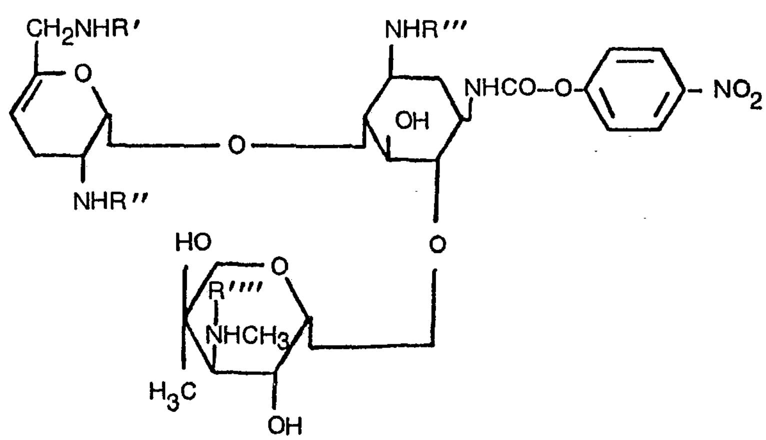

in which
R′, R″, R‴ and R″″ have the meaning mentioned in claim 3 are reacted with compounds of the formula

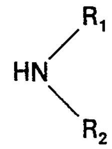

in which
$R_1$ and $R_2$ have the abovementioned meaning, the protective groups are split off and the desired products are isolated.

5. Process for the preparation of compounds according to claim 1, in which A denotes NH—$R_2$, characterised in that compounds of the formula

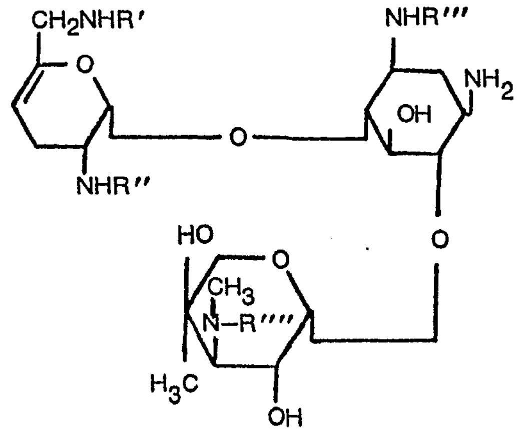

in which
R′, R″, R‴ and R″″ have the meaning mentioned in claim 3, are reacted with compounds of the formula

$R_2$—NCO

in which
$R_2$ has the meaning mentioned in claim 1, the protective groups are split off and the desired products are isolated.

6. Medicaments containing a compound according to claim 1.

7. Process for the preparation of a medicament, characterised in that a compound according to claim 1 is mixed with pharmaceutically suitable excipients and/or additives.